# EUROPEAN PATENT APPLICATION

(11) **EP 4 365 199 A1**
(43) Date of publication of application: **08.05.2024**
(21) Application number: 22831997.6
(22) Date of filing: 28.06.2022
(51) Int. Cl.: C07K 16/28, C07K 16/46, A61K 39/395, A61P 35/00, C12N 15/13, C12R 1/19, C12N 5/10, C12N 1/21, C12N 15/85

(54) **CD16 ANTIBODY AND USE THEREOF**

(30) Priority: 29.06.2021 CN 202110732732
(71) Applicant: Shandong Simcere Biopharmaceutical Co., Ltd., Shandong 264006 (CN)
(72) Inventor: WANG, Qiong, Shanghai 201318 (CN); FU, Yayuan, Shanghai 201318 (CN); YANG, Cuiqing, Shanghai 201318 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: Grünecker Patent- und Rechtsanwälte PartG mbB
(86) International application number: PCT/CN2022/101713
(87) International publication number: WO 2023/274183

(57) **Abstract**

The present invention relates to a CD16 antibody and the use thereof. Specifically, disclosed are an antibody or an antigen-binding fragment specifically binding CD16, and an encoding nucleic acid, an expression vector and an expression cell thereof, a preparation method therefor, a pharmaceutical composition thereof, and the use thereof for treating diseases, such as the use thereof in the treatment of tumours. The CD16 antibody holds great significance for the development of a CD16 antibody therapeutic drug and a CD16 detection reagent.

## Description

The present application claims the right of priority for Chinese patent application no. 202110732732.X, entitled "CD16 ANTIBODY AND USE THEREOF" and submitted to the China National Intellectual Property Administration on June 29, 2021. The above-mentioned prior application is incorporated into the present application by reference in its entirety.

### Technical Field

The present invention relates to the field of biomedicine, and specifically to a CD16 antibody and the use thereof.

### Background

Natural killer cell (NK cell) is a component of the innate immune system and account for approximately 5%-15% of circulating lymphocytes. Unlike B cells and T cells, NK cells do not express somatically rearranged antigen receptors, but instead express a series of activating and inhibitory receptors, and the integration and balance of activating and inhibitory signals from the interaction between ligands and different receptors determines the state of NK cell activation. Activated NK cells kill target cells by a similar means to cytotoxic T cells, namely by cytolytic granules containing perforin and granzymes and by a death receptor pathway. Activated NK cells also secrete inflammatory cytokines such as IFN-γ and chemokines that promote the recruitment of other inflammatory cells to target tissues. Unlike T cells, NK cells do not require antigen priming and recognize targets by activating receptors in the absence of MHC recognition.

Class I HLA (MHC I) molecules on the surface of tumour cells inhibit the activation of NK cells by binding to inhibitory receptors on the surface of NK cells. However, many tumour cells escape the surveillance of cytotoxic CD8⁺ T cells by downregulating the expression of MHC I molecules. Therefore, in the tumour microenvironment, when T cells fail to recognize tumour cells, NK cells can kill tumour cells through a "missing-self" mechanism due to the lack of MHC I-induced inhibitory signals.

The human IgG Fc receptor CD16 (FcγRIII) consists of two subtypes (CD16a/FcγRIIIa and CD16b/FcyRIIIb), and is encoded by two highly homologous genes. CD16b (FcyRIIIb), mainly expressed on neutrophils, is a GPI-anchored glycoprotein that lacks the intracellular signalling domain. There are gene polymorphisms in CD16b, which can produce three allotypes, namely NA1, NA2 and SH. CD16a, a low-affinity receptor for human IgG Fc, is a single transmembrane protein that is involved in antibody-dependent cellular cytotoxicity (ADCC) and triggers specific lysis of target cells by natural killer (NK) cells. ADCC is one of the dominant mechanisms of cytotoxicity by FcyR-expressing effector cells to clear tumour cells. A single nucleotide polymorphism (SNP) from G to T at position 559 of the nucleotide sequence in the cDNA of the CD16a gene generates two distinct FcyRIIIa allotypes: one encoded as valine (V), and the other encoded as phenylalanine (F), at position 158 of the amino acid sequence. The presence of valine (V/V or V/F) enhances the binding affinity of NK cells to IgG1 or IgG3 antibodies compared with a homozygous phenylalanine genotype (F/F), resulting in higher levels of NK cell-mediated ADCC. In an antibody-based immunotherapy, NK cell-mediated ADCC is one of the mechanisms of anticancer effects of commonly used antibodies such as rituximab, trastuzumab, and cetuximab. Analysis of several clinical studies demonstrates that in non-Hodgkin's lymphoma, HER-2/neu-positive metastatic breast cancer, metastatic colorectal cancer, or head and neck cancer, patients with the V/V polymorphism have improved progression-free survival compared with patients with the F/F polymorphism.

The ADCC function of NK cells has received high attention in an antibody immunotherapy. Bispecific antibodies that simultaneously recruit the ADCC receptor CD16a and recognize target antigens have been developed. Different formats of these bispecific antibodies are currently undergoing preclinical and clinical research, for example, GTB-3550 targeting CD33 and CD16, AFM24 targeting EGFR and CD16, and AFM26 targeting BCMA and CD16 are currently in clinical research, and the CD16 antibody moieties used in these antibodies are all derived from natural human phage display libraries. Nanobody (Nb) is a genetically engineered antibody containing only a single domain. In 1993, Belgian scientist Hamers-Casterman C discovered a natural heavy chain antibody in camel blood that only contained heavy chains but no light chains. Compared with a normal antibody, the heavy chain antibody has no light chains, but still retains the ability to bind to an antigen. After the variable region of the heavy chain antibody in camels is cloned, the obtained single domain antibody (sdAb) consisting of only one heavy chain variable region is called nanobody or VHH antibody (variable heavy chain domain of a heavy chain antibody). The nanobody not only has a molecular weight which is only 1/10 of that of a normal antibody, but also has more flexible chemical properties, good stability, high solubility, and high tumour tissue penetration, and is easily expressed.

In summary, the application of nanobody technology to develop a CD16-targeting therapeutic antibody that specifically recognizes CD16a and has comparable high binding affinity to the two allotypes of CD16a, has relatively low binding activity to CD16b, and is not affected by human immunoglobulin in serum, has broad prospects.

### Summary of the Invention

The present invention provides an antibody or an antigen-binding fragment specifically binding to CD16, a multi specific antigen-binding molecule, a chimeric antigen receptor, an immune effector cell, a nucleic acid fragment, a vector, a host cell, a pharmaceutical composition, a kit, a preparation method, and the use of the antibody or the antigen-binding fragment in the treatment of diseases and detection of CD 16.

In a first aspect, the present invention discloses an antibody or an antigen-binding fragment specifically binding to CD16, wherein the antibody or the antigen-binding fragment comprises CDR1, CDR2 and CDR3, and the CDR1, CDR2 and CDR3 comprise HCDR1, HCDR2 and HCDR3 selected from a VHH domain as shown in any one of SEQ ID NOs: 21-34 or SEQ ID NOs: 90-101, respectively.

In some specific embodiments, the HCDR1, HCDR2 and HCDR3 are determined according to the Kabat, Chothia or IMGT numbering system; optionally, the HCDR1, HCDR2 and HCDR3 are selected from Table 9; optionally, the HCDR1 is selected from SEQ ID NO: 49, 52, 54, 57, 60, 62, 65, 66, 67, 68, 70, 72, 78, 79, 80, 81 or 88; optionally, the HCDR2 is selected from SEQ ID NO: 50, 53, 55, 58, 61, 63, 69, 71, 73, 75, 76, 77, 82 or 85; optionally, the HCDR3 is selected from SEQ ID NO: 51, 56, 59, 64, 74, 83, 84, 86 or 87;
preferably, according to the Kabat, Chothia or IMGT numbering system, the HCDR1, HCDR2 and HCDR3 are selected from SEQ ID NOs: 49, 50 and 51, SEQ ID NOs: 52, 53 and 51 or SEQ ID NOs: 54, 55 and 56;
preferably, according to the Kabat, Chothia or IMGT numbering system, the HCDR1, HCDR2 and HCDR3 are selected from SEQ ID NOs: 57, 58 and 59, SEQ ID NOs: 60, 61 and 59 or SEQ ID NOs: 62, 63 and 64;
preferably, according to the Kabat, Chothia or IMGT numbering system, the HCDR1, HCDR2 and HCDR3 are selected from SEQ ID NOs: 49, 50 and 51, SEQ ID NOs: 65, 53 and 51 or SEQ ID NOs: 66, 55 and 56;
preferably, according to the Kabat, Chothia or IMGT numbering system, the HCDR1, HCDR2 and HCDR3 are selected from SEQ ID NOs: 49, 50 and 51, SEQ ID NOs: 67, 53 and 51 or SEQ ID NOs: 68, 55 and 56;
preferably, according to the Kabat, Chothia or IMGT numbering system, the HCDR1, HCDR2 and HCDR3 are selected from SEQ ID NOs: 49, 69 and 51, SEQ ID NOs: 70, 71 and 51 or SEQ ID NOs: 72, 73 and 74;
preferably, according to the Kabat, Chothia or IMGT numbering system, the HCDR1, HCDR2 and HCDR3 are selected from SEQ ID NOs: 49, 75 and 51, SEQ ID NOs: 52, 76 and 51 or SEQ ID NOs: 54, 77 and 56;
preferably, according to the Kabat, Chothia or IMGT numbering system, the HCDR1, HCDR2 and HCDR3 are selected from SEQ ID NOs: 49, 50 and 51, SEQ ID NOs: 78, 53 and 51 or SEQ ID NOs: 79, 55 and 56;
preferably, according to the Kabat, Chothia or IMGT numbering system, the HCDR1, HCDR2 and HCDR3 are selected from SEQ ID NOs: 49, 50 and 51, SEQ ID NOs: 80, 53 and 51 or SEQ ID NOs: 81, 55 and 56;
preferably, according to the Kabat, Chothia or IMGT numbering system, the HCDR1, HCDR2 and HCDR3 are selected from SEQ ID NOs: 49, 69 and 51, SEQ ID NOs: 65, 71 and 51 or SEQ ID NOs: 66, 73 and 56;
preferably, according to the Kabat, Chothia or IMGT numbering system, the HCDR1, HCDR2 and HCDR3 are selected from SEQ ID NOs: 57, 82 and 83, SEQ ID NOs: 60, 61 and 83 or SEQ ID NOs: 62, 63 and 84;
preferably, according to the Kabat, Chothia or IMGT numbering system, the HCDR1, HCDR2 and HCDR3 are selected from SEQ ID NOs: 57, 85 and 86, SEQ ID NOs: 60, 61 and 86 or SEQ ID NOs: 62, 63 and 87;
preferably, according to the Kabat, Chothia or IMGT numbering system, the HCDR1, HCDR2 and HCDR3 are selected from SEQ ID NOs: 57, 82 and 86, SEQ ID NOs: 60, 61 and 86 or SEQ ID NOs: 62, 63 and 87;
preferably, according to the Kabat, Chothia or IMGT numbering system, the HCDR1, HCDR2 and HCDR3 are selected from SEQ ID NOs: 88, 82 and 83, SEQ ID NOs: 60, 61 and 83 or SEQ ID NOs: 62, 63 and 84;
and preferably, according to the Kabat, Chothia or IMGT numbering system, the HCDR1, HCDR2 and HCDR3 are selected from SEQ ID NOs: 49, 50 and 51, SEQ ID NOs: 80, 53 and 51 or SEQ ID NOs: 81, 55 and 56.

In some specific embodiments, the CDR1, CDR2 and/or CDR3 comprise(s) an amino acid sequence with 1, 2 or 3 mutations on the HCDR1, HCDR2 and/or HCDR3; the mutations can be selected from insertion, deletion and/or substitution, and the substitution is preferably a conservative amino acid substitution.

In some specific embodiments, the CDR1, CDR2 and/or CDR3 comprise(s) a sequence having at least 80%, 85%, 90%, 91%, 92%, 93%, 94%, 95%, 96%, 97%, 98%, 99% or 100% identity to the HCDR1, HCDR2 and/or HCDR3.

In some specific embodiments, the antibody or the antigen-binding fragment comprises a single domain antibody, and the single domain antibody comprises the CDR1, CDR2 and CDR3.

In some specific embodiments, the single domain antibody comprises a sequence selected from as shown in any one of SEQ ID NOs: 21-34 or SEQ ID NOs: 90-101;
optionally, the single domain antibody comprises a sequence with at most 20, 19, 18, 17, 16, 15, 14, 13, 12, 11, 10, 9, 8, 7, 6, 5, 4, 3, 2 or 1 mutation relative to the sequence as shown in any one of SEQ ID NOs: 21-34 or SEQ ID NOs: 90-101, the mutation can be selected from insertion, deletion and/or substitution, and the substitution is preferably a conservative amino acid substitution;
and optionally, the single domain antibody comprises a sequence having at least 80%, 85%, 90%, 91%, 92%, 93%, 94%, 95%, 96%, 97%, 98%, 99% or 100% identity to the sequence as shown in any one of SEQ ID NOs: 21-34 or SEQ ID NOs: 90-101.

In some specific embodiments, the antibody comprises an FR region in a VHH domain as shown in any one of SEQ ID NOs: 21-34 or SEQ ID NOs: 90-101;
optionally, the antibody comprises a sequence with at most 15, 14, 13, 12, 11, 10, 9, 8, 7, 6, 5, 4, 3, 2 or 1 mutation relative to the FR region in the VHH domain as shown in any one of SEQ ID NOs: 21-34 or SEQ ID NOs: 90-101, the mutation can be selected from insertion, deletion and/or substitution, and the substitution is preferably a conservative amino acid substitution;
and optionally, the antibody comprises a sequence having at least 80%, 85%, 90%, 91%, 92%, 93%, 94%, 95%, 96%, 97%, 98%, 99% or 100% identity to the FR region in the VHH domain as shown in any one of SEQ ID NOs: 21-34 or SEQ ID NOs: 90-101.

In some specific embodiments, the antibody or the antigen-binding fragment is: (1) a chimeric antibody or a fragment thereof; (2) a humanized antibody or a fragment thereof; or (3) a fully human antibody or a fragment thereof.

In some specific embodiments, the antibody or the antigen-binding fragment comprises or does not comprise an antibody heavy chain constant region; optionally, the antibody heavy chain constant region can be selected from humans, llamas, mice, rats, rabbits or goats; optionally, the antibody heavy chain constant region can be selected from IgG, IgM, IgA, IgE or IgD, and the IgG can be selected from IgG1, IgG2, IgG3 or IgG4; optionally, the heavy chain constant region can be selected from an Fc region, a CH3 region or a complete heavy chain constant region; preferably, the heavy chain constant region is a human Fc region, and preferably comprises a sequence as shown in any one of SEQ ID NOs: 35-48 or SEQ ID NOs: 102-113; and preferably, the antibody or the antigen-binding fragment is a heavy chain antibody.

In some specific embodiments, the antibody or the antigen-binding fragment is further coupled with a therapeutic agent or a tracer; preferably, the therapeutic agent is selected from a radioisotope, a chemotherapeutic drug or an immunomodulator, and the tracer is selected from a radiological contrast agent, a paramagnetic ion, a metal, a fluorescent tag, a chemiluminescence tag, an ultrasonic contrast agent and a photosensitizer.

In some specific embodiments, the antibody or the antigen-binding fragment specifically binds to human CD16, cyno CD16 and/or murine CD16, and preferably, the KD of the antibody or the antigen-binding fragment to the human CD16 and/or the cyno CD16 is less than 1E-6M, 1E-7M, 2E-7M, 3E-7M, 4E-7M, 5E-7M, 6E-7M, 8E-7M, 9E-7M, 1E-8M, 2E-8M, 3E-8M, 4E-8M, 5E-8M, 6E-8M, 8E-8M, 9E-8M or 1E-9M.

In some specific embodiments, the antibody or the antigen-binding fragment binds to CD16A, and does not bind to CD16B or binds weakly to CD16B, and the CD16B is selected from CD16B (NA1), CD16B (NA2) or CD16B (SH).

In some specific embodiments, the antibody or the antigen-binding fragment is also linked to other functional molecules, and preferably, the other functional molecules can be selected from one or more of the following: a signal peptide, a protein tag, a cytokine, an angiogenesis inhibitor or an immune checkpoint inhibitor.

In some specific embodiments, the cytokine can be IL2, IL-6, IL-12, IL-15, IL-21, IFN or TNFα; the angiogenesis inhibitor can be endostatin; and the immune checkpoint inhibitor can be SIRPα.

In a second aspect, the present invention further discloses a multispecific antigen-binding molecule, wherein the multispecific antigen-binding molecule comprises the aforementioned antibody or antigen-binding fragment, and an antigen-binding molecule binding to an antigen other than CD16 or binding to a different CD16 epitope than that of the aforementioned antibody or antigen-binding fragment; optionally, the antigen other than CD16 can be selected from: CD137, CD258, PD-1, PD-L1, 4-1BB, CD40, CD64, EGFR, VEGF, HER2, HER1, HER3, IGF-1R, phosphatidylserine (PS), C-Met, BCMA, HSA, GPRC5D, MSLN, a blood brain barrier receptor, GPC3, PSMA, CD33, GD2, ROR1, ROR2, FRα or Gucy2C;
preferably, the other antigen-binding molecule is an antibody or an antigen-binding fragment;
preferably, the multispecific antigen-binding molecule can be bispecific, trispecific or tetraspecific;
and preferably, the multispecific antigen-binding molecule can be bivalent, tetravalent or hexavalent.

In a third aspect, the present invention further discloses an isolated nucleic acid fragment, wherein the nucleic acid fragment encodes the aforementioned antibody or antigen-binding fragment or multispecific antigen-binding molecule.

In a fourth aspect, the present invention further discloses a vector, wherein the vector comprises the aforementioned nucleic acid fragment.

In a fifth aspect, the present invention further discloses a host cell, wherein the host cell comprises the aforementioned vector; and preferably, the cell is a prokaryotic cell or a eukaryotic cell, such as a bacterial *(Escherichia coli*) cell, a fungal (yeast) cell, an insect cell or a mammalian cell (a CHO cell line or a 293T cell line).

In a sixth aspect, the present invention further discloses a method for preparing the aforementioned antibody or antigen-binding fragment or multispecific antigen-binding molecule, wherein the method comprises culturing the aforementioned cell, and isolating the antibody, antigen-binding fragment or multispecific antigen-binding molecule expressed by the cell.

In a seventh aspect, the present invention further discloses a pharmaceutical composition, wherein the pharmaceutical composition comprises the aforementioned antibody or antigen-binding fragment, multispecific antigen-binding molecule, nucleic acid fragment or vector, or a product prepared according to the aforementioned method; optionally, the pharmaceutical composition also comprises a pharmaceutically acceptable carrier, a diluent or an auxiliary agent; and optionally, the pharmaceutical composition further comprises an additional anti-tumour agent.

In an eighth aspect, the present invention further discloses a method for treating tumours or cancers, inflammatory diseases or allergies, wherein the method comprises administering to a subject an effective amount of the aforementioned antibody or antigen-binding fragment, multispecific antigen-binding molecule, nucleic acid fragment, vector, product prepared according to the aforementioned method or pharmaceutical composition; and preferably, the tumours or cancers are selected from non-Hodgkin's lymphoma, chronic lymphocytic leukaemia, Hodgkin disease, minimal residual disease and metastatic tumour.

In a ninth aspect, the present invention further discloses the aforementioned antibody or antigen-binding fragment, multispecific antigen-binding molecule, nucleic acid fragment, vector, product prepared according to the aforementioned method or pharmaceutical composition for use in the treatment of tumours or cancers, inflammatory diseases or allergies; preferably, the tumours or cancers are selected from non-Hodgkin's lymphoma, chronic lymphocytic leukaemia, Hodgkin disease, minimal residual disease and metastatic tumour.

In a tenth aspect, the present invention further discloses a kit, wherein the kit comprises the aforementioned antibody or antigen-binding fragment, multispecific antigen-binding molecule, nucleic acid fragment, vector, product prepared according to the aforementioned method or pharmaceutical composition.

In an eleventh aspect, the present invention further discloses a method for detecting CD16 expression in a biological sample, wherein the method comprises contacting the biological sample with the aforementioned antibody or antigen-binding fragment under conditions that allow the formation of a complex from the antibody or the antigen-binding fragment and CD16; and preferably, the method further comprises detecting the formation of the complex, thereby indicating the presence or expression level of CD16 in the sample.

In a twelfth aspect, the present invention further discloses the use of the aforementioned antibody or antigen-binding fragment in the preparation of a reagent for detecting CD16.

### Detailed Description of The Invention

### Definition and Description of Terminology

Unless otherwise defined herein, scientific and technical terms related to the present invention shall have the meanings understood by those of ordinary skill in the art.

In addition, unless otherwise specified herein, terms in the singular form herein shall include that in the plural form, and terms in the plural form shall include that in the singular form. More specifically, as used in the specification and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of".

The term "and/or" as used herein includes the meanings of "and", "or" and "all or any other combination of elements linked by the term".

The term "CD16" herein refers to a type III receptor for the Fc portion of immunoglobulin G (FcyRIII), a group of differentiation molecules found on the surface of natural killer cells, neutrophils, monocytes, and macrophages. CD16 has been identified as Fc receptors FcyRlIIa (CD16a) and FcyRlIIb (CD16b), which participate in signalling. Human FcγRIIIA (CD16a) is a low-affinity receptor that can bind to IgG Fc and is expressed on human CD56 low-expressing natural killer (NK) cells, monocyte subsets, dendritic cells and rare T cells. Human FcyRIIIB (CD16b), encoded by different genes and mainly expressed on neutrophils, is a GPI-anchored glycoprotein that lacks the intracellular signalling domain. CD16a is a type I membrane glycoprotein with a single transmembrane (TM) domain and a short cytoplasmic tail, whose expression on the cell surface depends on the binding to the signalling molecules CD247 (TCRζ) and/or Fc-εRI-γ; once they interact, a series of signallings are induced, leading to cytokine release and cell killing activity.

The term "specifically bind" herein means that an antigen-binding molecule (e.g., an antibody) typically specifically binds to an antigen and substantially the same antigen with a high affinity, but does not bind to an unrelated antigen with a high affinity. Affinity is typically reflected by the equilibrium dissociation constant (KD), where lower KD indicates higher affinity. Taking an antibody as an example, a high affinity generally means having a KD of about 10⁻⁶ M or less, 10⁻⁷ M or less, about 10⁻⁸ M or less or about 10⁻⁹ M or less. KD is calculated as follows: KD = Kd/Ka, where Kd represents the dissociation rate and Ka represents the binding rate. The equilibrium dissociation constant KD can be measured using well-known methods in the art, such as surface plasmon resonance (e.g., Biacore) or equilibrium dialysis assay.

The term "antigen-binding molecule" is used herein in the broadest sense and refers to a molecule specifically binding to an antigen. Exemplarily, the antigen-binding molecule includes, but is not limited to, an antibody or an antibody mimetic. "Antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. "Antibody" herein encompasses various forms and various structures as long as they exhibit the desired antigen-binding activity. "Antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, for example, Korndorfer, I. P., Beste, G. & Skerra, A. (2003). Proteins, 53, 121-129.; Roque, A. C. A., Lowe, C. R. & Taipa, M. A. Antibodies and genetically engineered related molecules: production and purification. Biotechnol. Prog. 20, 639-654 (2004). Such scaffolds may also include non-antibody-derived scaffolds, for example, scaffold proteins known in the art which can be used for grafting CDRs, including but not limited to tenascin, fibronectin, a peptide aptamer, *etc.*

The term "antibody" includes an intact antibody and any antigen-binding fragment (i.e., "antigen-binding portion") or single chain thereof. "Antibody" refers to a glycoprotein comprising at least two heavy (H) chains and two light (L) chains interconnected by a disulphide bond, or an antigen-binding portion thereof. Each heavy chain consists of a heavy chain variable region (abbreviated herein as VH) and a heavy chain constant region. The heavy chain constant region consists of three domains, CH1, CH2 and CH3. Each light chain consists of a light chain variable region (abbreviated herein as VL) and a light chain constant region. The light chain constant region consists of one domain, CL. The VH and VL regions can be further subdivided into hypervariable regions known as complementarity determining regions (CDRs), which are interspersed among more conserved regions known as framework regions (FRs). Each VH and VL consists of three CDRs and four FRs, which are arranged in the following order from amino terminus to carboxyl terminus: FR1, CDR1, FR2, CDR2, FR3, CDR3, FR4. The variable regions of the heavy and light chains contain a binding domain that can interact with an antigen. The constant region of an antibody can mediate the binding of an immunoglobulin to a host tissue or factor, and the host tissue or factor includes various cells of the immune system (e.g., effector cells) and a first component of the classical complement system (C1q). Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA, and IgE, and the corresponding heavy chains thereof are a mu (µ) chain, a delta (δ) chain, a gamma (γ) chain, an alpha (α) chain and an epsilon (ε) chain, respectively. The same class of Ig can also be divided into different subclasses according to the differences in the amino acid composition of the hinge region thereof and the number and position of heavy chain disulphide bonds. For example, IgG can be divided into IgG1, IgG2, IgG3 and IgG4, and IgA can be divided into IgA1 and IgA2. The light chains are divided into a kappa chain or a lambda chain by the difference in the constant region. Each Ig class of the five Ig classes can have either a kappa (κ) chain or a lambda (λ) chain.

"Antibody" herein also includes an antibody that does not comprise light chains, for example, heavy-chain antibodies (HCAbs) produced from camelids, such as Camelus dromedarius, Camelus bactrianus, Lama glama, Lama guanicoe and Vicugna pacos, and Ig new antigen receptors (IgNARs) found in Chondrichthyes such as sharks.

The term "antibody" herein can be derived from any animals, including but not limited to humans and non-human animals. The non-human animals can be selected from primates, mammals, rodents and vertebrates, such as camelids, Lama glama, Lama guanicoe, Vicugna pacos, goats, rabbits, mice, rats or Chondrichthyes (such as sharks).

The term "heavy chain antibody" herein refers to an antibody that lacks light chains of a conventional antibody. The term specifically includes, but is not limited to, a homodimeric antibody comprising a VH antigen-binding domain and CH2 and CH3 constant domains in the absence of a CH1 domain.

The term "nanobody" herein refers to a natural heavy chain antibody without light chains in camel, *etc.,* and cloning the variable region thereof can obtain a single domain antibody, also known as VHH (Variable domain of heavy chain of heavy chain antibody), which only consists of a heavy chain variable region, and is the smallest functional antigen-binding fragment.

The terms "VHH domain", "nanobody" and "single domain antibody" (sdAb) herein have the same meaning and are used interchangeably, and refer to a single domain antibody consisting of only one heavy chain variable region constructed by cloning the variable region of a heavy chain antibody, which is the smallest antigen-binding fragment with full functionality. Typically, a single domain antibody consisting of only one heavy chain variable region is constructed by obtaining a heavy chain antibody that naturally lacks light chains and heavy chain constant region 1 (CH1), and then cloning the heavy chain variable region of the antibody.

Further descriptions of "heavy chain antibody" and "single domain antibody", and "VHH domain" and "nanobody" can be found in Hamers-Casterman et al., Nature. 363, 446-8 (1993). Naturally occurring antibodies devoid of light chains.; the review article by Muyldermans (J Biotechnol. 2001 Jun; 74(4):277-302. Single domain camel antibodies: current status); and the following patent applications mentioned as general background art: WO 94/04678, WO 95/04079 and WO 96/34103; WO 94/25591, WO 99/37681, WO 00/40968, WO 00/43507, WO 00/65057, WO 01/40310, WO 01/44301, EP 1134231 and WO 02/48193; WO 97/49805, WO 01/21817, WO 03/035694, WO 03/054016 and WO 03/055527; WO 03/050531; WO 01/90190; WO 03/025020; and WO 04/041867, WO 04/041862, WO 04/041865, WO 04/041863, WO 04/062551, WO 05/044858, WO 06/40153, WO 06/079372, WO 06/122786, WO 06/122787 and WO 06/122825, and other prior art mentioned in these applications.

The term "multispecific" herein refers to the ability of an antibody or an antigen-binding fragment to bind to, for example, different antigens or at least two different epitopes on the same antigen. Therefore, the terms such as "bispecific", "trispecific", and "tetraspecific" refer to the number of different epitopes to which an antibody can bind. For example, a conventional monospecific IgG-type antibody has two identical antigen-binding sites (paratopes) and thus can only bind to the same epitope (rather than bind to different epitopes). In contrast, a multispecific antibody has at least two different types of paratopes/binding sites and thus can bind to at least two different epitopes. As described herein, "complementarity determining region" refers to an antigen-binding site of an antibody. Furthermore, single "specific" may refer to one, two, three or more identical complementarity determining regions in a single antibody (the actual number of complementarity determining regions/binding sites in a single antibody molecule is referred to as "valent"). For example, a single natural IgG antibody is monospecific and bivalent because it has two identical paratopes. Accordingly, a multispecific antibody comprises at least two (different) complementarity determining regions/binding sites. Therefore, the term "multispecific antibody" refers to an antibody that has more than one paratope and has the ability to bind to two or more different epitopes. The term "multispecific antibody" particularly includes the bispecific antibody as defined above, and generally also includes a protein, e.g., an antibody or a scaffold specifically binding to three or more different epitopes, *i.e.,* an antibody having three or more paratopes/binding sites.

The term "valent" herein refers to the presence of a defined number of binding sites in an antibody/antigen-binding molecule. Therefore, the terms "monovalent", "bivalent", "tetravalent" and "hexavalent" indicate the presence of one binding site, two binding sites, four binding sites and six binding sites in an antibody/antigen-binding molecule, respectively.

"Full-length antibody", "complete antibody" and "intact antibody" are used interchangeably herein and mean that they have a structure substantially similar to that of a natural antibody.

"Antigen-binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. Exemplarily, "antigen-binding fragment" or "antibody fragment" herein includes, but is not limited to, Fab, F(ab')2, Fab', Fab'-SH, Fd, Fv, scFv, diabody and a single domain antibody.

The term "chimeric antibody" herein refers to an antibody that has a variable sequence of an immunoglobulin from a source organism (such as a rat, a mouse, a rabbit or a llama) and a constant region of an immunoglobulin from a different organism (such as human). Methods for producing the chimeric antibody are known in the art. See, for example, Morrison, 1985, Science 229(4719): 1202-1207. Transfectomas Provide Novel Chimeric Antibodies; and Gillies et al., J Immunol Methods. 1989 Dec 20; 125(1-2): 191-202; the above documents are incorporated herein by reference. See also patent US 5807715 A.

The term "humanized antibody" herein refers to a non-human antibody that has been genetically engineered, with amino acid sequences modified to improve the homology with the sequences of a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody usually retains or partially retains the expected properties of a donor antibody, including but not limited to, antigen specificity, affinity, reactivity, ability to enhance immune cell activity, ability to enhance immune response, *etc.*

The term "fully human antibody" herein refers to an antibody having variable regions in which both the FRs and CDRs are derived from human germline immunoglobulin sequences. Furthermore, if the antibody comprises a constant region, the constant region is also derived from human germline immunoglobulin sequences. Fully human antibody herein may include amino acid residues not encoded by human germline immunoglobulin sequences (for example, mutations introduced by random or site-specific mutagenesis *in vitro* or by somatic mutation *in vivo).* However, "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains of a natural antibody generally have similar structures, and each domain comprises four conserved framework regions (FRs) and three hypervariable regions (HVRs). A single VH or VL domain may be sufficient to confer antigen-binding specificity.

The terms "complementarity determining region" and "CDR" are used interchangeably herein and generally refer to hypervariable regions (HVRs) found in both light and heavy chain variable domains. The more conserved portions in variable domains are called the framework regions (FRs). As understood in the art, the amino acid positions representing the hypervariable regions of an antibody can vary according to the context and various definitions known in the art. Some positions within variable domains can be considered heterozygous hypervariable positions because these positions can be considered to be within the hypervariable regions under one set of criteria (such as IMGT or KABAT) but outside the hypervariable regions under a different set of criteria (such as KABAT or IMGT). One or more of these positions may also be found in extended hypervariable regions. The present invention includes an antibody comprising modifications in these heterozygous hypervariable positions. The heavy chain variable region CDR can be abbreviated as HCDR, and the light chain variable region CDR can be abbreviated as LCDR. The variable domains of natural heavy and light chains respectively comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in the order of FR1-CDR1-FR2-CDR2-FR3-CDR3-FR4 by the FR region, and together with the CDRs from other antibody chains, contribute to the formation of an antigen-binding site of an antibody.

For a further description of CDRs, see Kabat, E. A., Wu, T. T., & Bilofsky, H. (1977). Unusual distributions of amino acids in complementarity determining (hypervariable) segments of heavy and light chains of immunoglobulins and their possible roles in specificity of antibody-combining sites. Journal of Biological Chemistry, 252(19), 6609-6616.; Kabat et al., United States Department of Health and Human Services, "Sequences of proteins of immunological interest" (1991); Chothia, C., & Lesk, A. M. (1987). Canonical structures for the hypervariable regions of immunoglobulins. Journal of molecular biology, 196(4), 901-917.; Al-Lazikani, B., Lesk, A. M., & Chothia, C. (1997). Standard conformations for the canonical structures of immunoglobulins. Journal of molecular biology, 273(4), 927-948.; MacCallum, R. M., Martin, A. C., & Thornton, J. M. (1996). Antibody-antigen interactions: contact analysis and binding site topography. Journal of molecular biology, 262(5), 732-745.; Abhinandan, K. R., & Martin, A. C. (2008). Analysis and improvements to Kabat and structurally correct numbering of antibody variable domains. Molecular immunology, 45(14), 3832-3839.; Lefranc, M. P., Pommié, C., Ruiz, M., Giudicelli, V., Foulquier, E., Truong, L., ... & Lefranc, G. (2003). IMGT unique numbering for immunoglobulin and T cell receptor variable domains and Ig superfamily V-like domains. Developmental & Comparative Immunology, 27(1), 55-77.; and Honegger, A., & PluÈckthun, A. (2001). Yet another numbering scheme for immunoglobulin variable domains: an automatic modelling and analysis tool. Journal of molecular biology, 309(3), 657-670. The "CDRs" herein can be marked and defined by well-known methods in the art, including but not limited to Kabat numbering system, Chothia numbering system or IMGT numbering system. The tool websites used include, but are not limited to, AbRSA website (http://cao.labshare.cn/AbRSA/cdrs.php), abYsis web site (www.abysis.org/abysis/sequence_input/key_annotation/key_annotation.cgi) and IMGT website (http://www.imgt.org/3Dstructure-DB/cgi/DomainGapAlign.cgi# results). The CDRs herein include overlaps and subsets of amino acid residues defined in different ways.

The term "Kabat numbering system" herein generally refers to the immunoglobulin alignment and numbering system proposed by Elvin A. Kabat (see, for example, Kabat et al., Sequences of Proteins of Immunological Interest, 5th Ed. Public Health Service, National Institutes of Health, Bethesda, Md., 1991).

The term "Chothia numbering system" herein generally refers to the immunoglobulin numbering system proposed by Chothia *et al.,* which is a classical rule for identifying the boundaries of CDR regions based on the location of structural loop regions (see, for example, Chothia, C., & Lesk, A. M. (1987). Canonical structures for the hypervariable regions of immunoglobulins. Journal of molecular biology, 196(4), 901-917.).

The term "IMGT numbering system" herein generally refers to the numbering system based on the international ImMunoGeneTics information system (IMGT) initiated by Lefranc et al., see Lefranc, M. P., Pommié, C., Ruiz, M., Giudicelli, V., Foulquier, E., Truong, L., ... & Lefranc, G. (2003). IMGT unique numbering for immunoglobulin and T cell receptor variable domains and Ig superfamily V-like domains. Developmental & Comparative Immunology, 27(1), 55-77.

The term "heavy chain constant region" herein refers to the carboxyl-terminal portion of the heavy chain of an antibody, which is not directly involved in the binding of the antibody to an antigen, but exhibits effector functions, such as interaction with Fc receptors, and has a more conserved amino acid sequence relative to the variable domain of the antibody. "Heavy chain constant region" can be selected from a CH1 domain, a hinge region, a CH2 domain, a CH3 domain, or a variant or fragment thereof. "Heavy chain constant region" includes "full-length heavy chain constant region" and "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain; when the antibody is IgE, it also includes a CH4 domain; and when the antibody is a heavy chain antibody, it does not include the CH1 domain. Exemplarily, a typical "heavy chain constant region fragment" can be selected from Fc or a CH3 domain.

The term "light chain constant region" herein refers to the carboxyl-terminal portion of the light chain of an antibody, which is not directly involved in the binding of the antibody to an antigen. The light chain constant region can be selected from a constant kappa domain or a constant lambda domain.

The term "Fc region" herein is used to define the C-terminal region of an antibody heavy chain that contains at least a portion of the constant region. The term includes a native sequence Fc region and a variant Fc region. Exemplarily, human IgG heavy chain Fc region can extend from Cys226 or Pro230 to the carboxyl terminus of the heavy chain. However, an antibody generated from a host cell may undergo post-translational cleavage whereby one or more, particularly one or two amino acids are cleaved off from the C-terminus of the heavy chain. Therefore, by expression of a specific nucleic acid molecule encoding a full-length heavy chain, the antibody generated from a host cell can include the full-length heavy chain, or a cleavage variant of the full-length heavy chain. This may be the case when the last two C-terminal amino acids of the heavy chain are glycine (G446) and lysine (K447, numbering according to the Kabat EU index). Therefore, the C-terminal lysine (Lys447), or the C-terminal glycine (Gly446) and lysine (Lys447) of the Fc region may or may not be present. Typically, an IgG Fc region comprises IgG CH2 and IgG CH3 domains, and optionally, on this basis, the IgG Fc region can also comprise a complete or partial hinge region, but does not comprise a CH1 domain. The "CH2 domain" of a human IgG Fc region generally extends from an amino acid residue at about position 231 to an amino acid residue at about position 340. In one embodiment, a carbohydrate chain is attached to the CH2 domain. The CH2 domain herein can be a native sequence CH2 domain or a variant CH2 domain. "CH3 domain" comprises the stretch of residues at the C-terminus of the CH2 domain in the Fc region (i.e., from an amino acid residue at about position 341 to an amino acid residue at about position 447 of IgG). The CH3 region herein can be a native sequence CH3 domain or a variant CH3 domain (e.g., a CH3 domain having a "knob" introduced in one chain thereof and a "hole" correspondingly introduced in the other chain thereof; see US Patent No.5,821,333, which is expressly incorporated herein by reference). As described herein, such variant CH3 domains can be used to facilitate heterodimerization of two non-identical antibody heavy chains.

Unless otherwise specified herein, the numbering of amino acid residues in an Fc region or a constant region is according to the EU numbering system, also known as the EU index, as described in Kabat et al., Sequences of Proteins of Immunological Interest, 5th Ed. Public Health Service, National Institutes of Health, Bethesda, MD, 1991.

The term "Fc variant" herein refers to changes in the structure or function of Fc caused by one or more amino acid substitution, insertion or deletion mutations at appropriate positions on the Fc. "Interaction between Fc variants" refers to the space-filling effect, electrostatic steering, hydrogen bonding effect, hydrophobic effect, *etc.* formed between Fc variants that have been subjected to a mutation design. The interaction between Fc variants contributes to the formation of a stable heterodimeric protein. A preferred mutation design is a mutation design in a "Knob-into-Hole" form.

The mutation design technique of Fc variants has been widely used in the field to prepare bispecific antibodies or heterodimeric Fc fusion protein forms. Representative examples include a "Knob-into-Hole" form proposed by Cater et al. (Ridgway, J. B., Presta, L. G., & Carter, P. (1996). 'Knobs-into-holes' engineering of antibody CH3 domains for heavy chain heterodimerization. Protein Engineering, Design and Selection, 9(7), 617-621.); an Fc-containing heterodimer form formed by using Electrostatic Steering by the technician in Amgen (US 20100286374 A1); a heterodimer form formed by IgG/IgA strand exchange (SEEDbodies) proposed by Jonathan H.Davis et al. (Davis, J. H., Aperlo, C., Li, Y., Kurosawa, E., Lan, Y., Lo, K. M., & Huston, J. S. (2010). SEEDbodies: fusion proteins based on strand-exchange engineered domain (SEED) CH3 heterodimers in an Fc analogue platform for asymmetric binders or immunofusions and bispecific antibodies. Protein Engineering, Design & Selection, 23(4), 195-202.); a bispecific molecule formed by the platform technique DuoBody of Genmab (Gramer, M. J., van den Bremer, E. T., van Kampen, M. D., Kundu, A., Kopfmann, P., Etter, E., ... & Parren, P. W. (2013, November). Production of stable bispecific IgG1 by controlled Fab-arm exchange: scalability from bench to large-scale manufacturing by application of standard approaches. In MAbs (Vol. 5, No. 6, pp. 962-973). Taylor & Francis.); a heterodimeric protein form formed by combining structural calculations, Fc amino acid mutations, and different modes of action by the technician in Xencorto (Moore, G. L., Bautista, C., Pong, E., Nguyen, D. H. T., Jacinto, J., Eivazi, A., ... & Lazar, G. A. (2011, November). A novel bispecific antibody format enables simultaneous bivalent and monovalent co-engagement of distinct target antigens. In MAbs (Vol. 3, No. 6, pp. 546-557). Taylor & Francis.); a heterodimeric protein form obtained by the charge network-based Fc modification method of Alphamab Oncology (CN 201110459100.7); and other genetic engineering methods for forming heterodimeric functional proteins based on Fc amino acid changes or functional modification means. The Knob/Hole structure on the Fc variant fragments of the present invention means that the two Fc fragments are mutated respectively, and can be combined in a "Knob-into-Hole" form after the mutations. Preferably, the Fc regions are subjected to site mutation modification using the "knob-into-hole" model of Cater *et al.,* so that the obtained first Fc variant and second Fc variant can be combined together in a "knob-into-hole" form to form a heterodimer. The selection of particular immunoglobulin Fc regions from particular immunoglobulin classes and subclasses is within the range of those skilled in the art. The Fc regions of human antibodies IgG1, IgG2, IgG3 and IgG4 are preferred, and the Fc region of human antibody IgG1 is more preferred. Either of the first Fc variant or the second Fc variant is randomly selected for knob mutation and the other for hole mutation.

The term "conservative amino acid" herein generally refers to amino acids that belong to the same class or have similar characteristics (e.g., charge, side chain size, hydrophobicity, hydrophilicity, backbone conformation, and rigidity). Exemplarily, the following amino acids in each group are each other's conservative amino acid residues, and a substitution of amino acid residues in the group is a conservative amino acid substitution:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method. To determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position. The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needlema and Wunsch algorithm in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70, or 80 and a length weight of 1, 2, 3, 4, 5, or 6. A particularly preferred parameter set (and one parameter set that should be used unless otherwise stated) is the Blossum62 scoring matrix with a gap penalty of 12, a gap extension penalty of 4, and a gap frameshift penalty of 5. The percent identity between two amino acid sequences or nucleotide sequences can also be determined using the PAM120 weighted remainder table, with a gap length penalty of 12 and a gap penalty of 4, and using the E. Meyers and W. Miller algorithm that has been incorporated into the ALIGN program (version 2.0).

Additionally or alternatively, the nucleic acid and protein sequences of the present invention can further be used as "query sequences" to perform searches against public databases, e.g., to identify other family member sequences or related sequences. For example, such searches can be performed using the NBLAST and XBLAST programs (version 2.0) in Altschul, S. F., Gish, W., Miller, W., Myers, E. W., & Lipman, D. J. (1990). Basic local alignment search tool. Journal of molecular biology, 215(3), 403-410. BLAST nucleotide searches can be performed with the NBLAST program, with score = 100 and word length = 12, to obtain the nucleotide sequences homologous to the nucleic acid molecules of the present invention. BLAST protein searches can be performed with the XBLAST program, with score = 50 and word length = 3, to obtain the amino acid sequences homologous to the protein molecules of the present invention. To obtain gapped alignment results for comparison purposes, gapped BLAST can be used as described in Altschul, S. F., Madden, T. L., Schäffer, A. A., Zhang, J., Zhang, Z., Miller, W., & Lipman, D. J. (1997). Gapped BLAST and PSI-BLAST: a new generation of protein database search programs. Nucleic acids research, 25(17), 3389-3402. When BLAST and gapped BLAST programs are used, the default parameters of the corresponding programs (e.g., XBLAST and NBLAST) can be used. See www.ncbi.nlm.nih.gov.

The term "chimeric antigen receptor (CAR)" herein refers to an artificial cell surface receptor engineered to express on immune effector cells and specifically bind to antigens, comprising at least (1) an extracellular antigen-binding domain, such as a heavy chain variable region and/or a light chain variable region of an antibody, (2) a transmembrane domain anchoring the CAR into an immune effector cell, and (3) an intracellular signalling domain. The CAR can redirect T cells and other immune effector cells to selected targets, such as cancer cells, in a non-MHC-restricted way by using an extracellular antigen-binding domain.

The term "nucleic acid" herein includes any compound and/or substance comprising a polymer of nucleotides. Each nucleotide consists of a base, specifically a purine or pyrimidine base *(i.e.,* cytosine (C), guanine (G), adenine (A), thymine (T) or uracil (U)), a sugar *(i.e.,* deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of the non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the present invention *in vitro* and/or *in vivo,* for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody *in vivo* (see, for example, Stadler, C. R., Bähr-Mahmud, H., Celik, L., Hebich, B., Roth, A. S., Roth, R. P., ... & Sahin, U. (2017). Elimination of large tumours in mice by mRNA-encoded bispecific antibodies. Nature medicine, 23(7), 815-817. or EP 2101823 B1).

"Isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of the nucleic acid to which they are operably linked. Such vectors are referred to herein as "expression vectors".

The term "host cell" herein refers to a cell into which a foreign nucleic acid is introduced, including the progeny of such a cell. The host cell includes a "transformant" and a "transformed cell" which include a primary transformed cell and the progeny derived therefrom, regardless of the number of passages. The progeny may not be identical to the parental cell in nucleic acid content, and may contain a mutation. The mutant progenies with the same function or biological activity as those screened or selected in the initially transformed cells are included herein.

The term "pharmaceutical composition" herein refers to a preparation that is present in a form which allows the active ingredients contained therein to be biologically effective and does not contain additional ingredients that would be unacceptably toxic to a subject to which the pharmaceutical composition is administered.

The term "pharmaceutically acceptable carrier" herein includes any and all solvents, dispersion media, coating materials, surfactants, antioxidants, preservatives (e.g., antibacterial agents and antifungal agents), isotonic agents, absorption delaying agents, salts, preservatives, drug stabilizers, binders, excipients, disintegrants, lubricants, sweeteners, flavouring agents, dyes, *etc.,* and a combination thereof, which are known to those skilled in the art (see, for example, Remington's Pharmaceutical Sciences, 18th edition. MackPrinting Company, 1990, pages 1289-1329). Except in cases of incompatibility with the active ingredient, any conventional carrier is contemplated for use in a therapeutic or pharmaceutical composition.

The term "treatment" herein refers to surgical or therapeutic treatment, the purpose of which is to prevent and slow down (reduce) an undesired physiological change or pathology in a subject being treated, such as cancers and tumours. Beneficial or desired clinical results include, but are not limited to, alleviation of symptoms, diminishment of extent of disease, stabilized *(i.e.,* not worsening) state of disease, delay or slowing of disease progression, amelioration or palliation of the disease state and remission (whether partial response or complete response), whether detectable or undetectable. Subjects in need of treatment include subjects who already have a condition or a disease, subjects who are prone to a condition or a disease or subjects who intend to prevent a condition or a disease. When referring to terms such as slow down, alleviation, diminishment, palliation, and remission, the meaning of elimination, disappearance, non-occurrence, *etc.* is also included.

The term "subject" refers to an organism receiving treatment for a particular disease or condition as described herein. Exemplarily, a "subject" includes a mammal, such as a human, a primate (e.g., monkey) or a non-primate mammal, receiving treatment for a disease or a condition.

The term "effective amount" herein refers to an amount of a therapeutic agent effective to prevent or relieve a disease or a condition or the progression of the disease when administered alone or in combination with another therapeutic agent to a cell, tissue or subject. "Effective amount" also refers to an amount of a compound sufficient to relieve symptoms, for example, treat, cure, prevent or relieve the associated medical conditions, or to increase the rate of treating, curing, preventing or relieving these conditions. When an active ingredient is administered to an individual alone, a therapeutically effective dose refers to the ingredient alone. When a combination is used, a therapeutically effective dose refers to the combined amounts of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumour" or "neoplasm" herein refers to all neoplastic cell growth and proliferation, whether malignant or benign, and to all pre-cancerous and cancerous cells and tissues. The terms "cancer" and "tumour" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Brief Description of the Figures

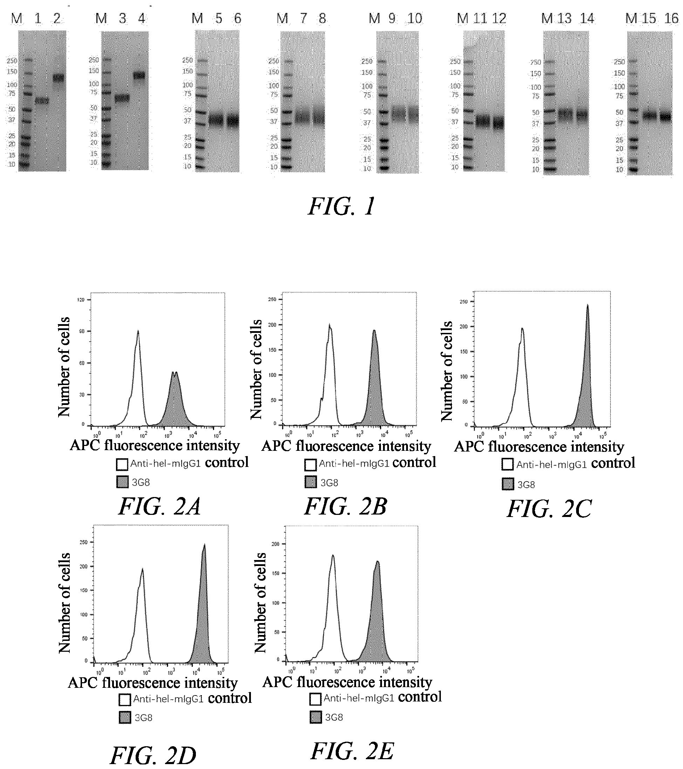

FIG. 1 shows the detection results of SDS-PAGE reducing and non-reducing gels of CD16a (158F)-Fc, human CD16a (158V)-Fc, CD16a (158F)-his, human CD16a (158V)-his, human CD16b (NA1)-his, human CD16b (NA2)-his, human CD16b (SH)-his and cynomolgus monkey CD16-his protein samples. Lanes 1 and 2 are the protein bands of human CD16a (158F)-Fc under the reducing and non-reducing conditions, respectively; lanes 3 and 4 are the protein bands of human CD16a (158V)-Fc under the reducing and non-reducing conditions, respectively; lanes 5 and 6 are the protein bands of human CD16a (158F)-his under the reducing and non-reducing conditions, respectively; lanes 7 and 8 are the protein bands of human CD16a (158V)-his under the reducing and non-reducing conditions, respectively; lanes 9 and 10 are the protein bands of human CD16b (NA1)-his under the reducing and non-reducing conditions, respectively; lanes 11 and 12 are the protein bands of human CD16b (NA2)-his under the reducing and non-reducing conditions, respectively; lanes 13 and 14 are the protein bands of human CD16b (SH)-his under the reducing and non-reducing conditions, respectively; lanes 15 and 16 are the protein bands of cynomolgus monkey CD16-his under the reducing and non-reducing conditions, respectively; and lane M is the protein maker band.
FIG. 2A shows the FACS detection results of FlpinCHO cells transfected with a human CD16a (158F) protein.
FIG. 2B shows the FACS detection results of FlpinCHO cells transfected with a human CD16a (158V) protein.
FIG. 2C shows the FACS detection results of FlpinCHO cells transfected with a human CD16b (NA1) protein.
FIG. 2D shows the FACS detection results of FlpinCHO cells transfected with a human CD16b (NA2) protein.
FIG. 2E shows the FACS detection results of FlpinCHO cells transfected with a cynomolgus monkey CD16 protein.
FIG. 3A shows the binding activity of CD16 VHH-Fc antibodies to FlpinCHO-human CD16a (158F) cells.
FIG. 3B shows the binding activity of CD16 VHH-Fc antibodies to FlpinCHO-human CD16a (158V) cells.
FIG. 4A shows the binding activity of biotinylated CD16 VHH-Fc antibodies to FlpinCHO-human CD16a (158V) cells.
FIG. 4B shows the binding activity of biotinylated CD16 VHH-Fc antibodies to FlpinCHO-human CD16a (158V) cells in the presence of 10 mg/mL human immunoglobulin.
FIG. 4C shows the binding activity of biotinylated CD16 VHH-Fc antibodies to FlpinCHO-human CD16a (158F) cells.
FIG. 4D shows the binding activity of biotinylated CD16 VHH-Fc antibodies to FlpinCHO-human CD16a (158F) cells in the presence of 10 mg/mL human immunoglobulin.
FIG. 5A shows the binding activity of CD16 VHH-Fc antibodies with FlpinCHO-human CD16b (NA1) cells detected by FACS.
FIG. 5B shows the binding activity of CD16 VHH-Fc antibodies with FlpinCHO-human CD16b (NA2) cells detected by FACS.
FIG. 6A shows the binding activity of CD16 VHH-Fc antibodies to human NK cells.
FIG. 6B shows the binding activity of biotinylated CD16 VHH-Fc antibodies to human NK cells.
FIG. 6C shows the binding activity of biotinylated CD16 VHH-Fc antibodies to human NK cells in the presence of 10 mg/mL human immunoglobulin.
FIG. 7 shows the binding activity of CD16 VHH-Fc antibodies with FlpinCHO-cyno CD16 cells detected by FACS.
FIG. 8 shows the activation of Jurkat-NFAT cells by antibodies detected by a luciferase reporter system.
FIG. 9A shows the binding activity of humanized CD16 VHH-Fc antibodies to FlpinCHO-human CD16a (158F) cells.
FIG. 9B shows the binding activity of humanized CD16 VHH-Fc antibodies to FlpinCHO-human CD16a (158V) cells.
FIG. 10A shows the binding activity of humanized CD16 VHH-Fc antibodies with FlpinCHO-human CD16b (NA1) cells.
FIG. 10B shows the binding activity of humanized CD16 VHH-Fc antibodies with FlpinCHO-human CD16b (NA2) cells.
FIG. 11A shows the binding activity of biotinylated CD16 VHH-Fc antibodies to NK cells.
FIG. 11B shows the binding activity of biotinylated CD16 VHH-Fc antibodies to NK cells in the presence of 10 mg/mL human immunoglobulin.
FIG. 12 shows the binding of humanized CD16 VHH-Fc antibodies to human neutrophils.
FIG. 13 shows the binding activity of humanized CD16 VHH-Fc antibodies with FlpinCHO-cyno CD16 cells.
FIG. 14 shows the activation of Jurkat-NFAT cells by humanized CD16 VHH-Fc antibodies detected by a luciferase reporter system.

### Examples

The present invention will be further described below in conjunction with specific examples, and the advantages and characteristics of the present invention will become clearer along with the description. If specific conditions are not specified in the examples, conventional conditions or conditions recommended by a manufacturer are followed. The reagents or instruments used therein for which manufacturers are not specified are all conventional products that are commercially available.

The examples of the present invention are merely exemplary, and do not limit the scope of the present invention in any way. Those skilled in the art should understand that the details and forms of the technical solutions of the present invention can be modified or replaced without departing from the spirit and scope of the present invention, but these modifications and replacements all fall within the scope of protection of the present invention.

### Example 1 Preparation of CD16-related antigen and antibody and construction of stably transfected cell line

### 1.1 Preparation of human and cynomolgus monkey CD 16 antigens

The full-length amino acid sequence of human CD16a (158F) (Uniprot ID: P08637, SEQ ID NO: 1), full-length amino acid sequence of human CD16a (158V) (NCBI ID: AAH17865.1, SEQ ID NO: 2), full-length amino acid sequence of human CD16b (NA1) (NCBI ID: AAA35881.1, SEQ ID NO: 3), full-length amino acid sequence of human CD16b (NA2) (Uniprot ID: 075015, SEQ ID NO: 4) and full-length amino acid sequence of human CD16b (SH) (SEQ ID NO: 5) from patent WO-2016177846, and the full-length amino acid sequence of cynomolgus monkey CD16 (NCBI ID: NP_001270121.1, SEQ ID NO: 6) were as shown in Table 1. The extracellular domains (ECDs) of the above-mentioned proteins were sequentially linked to human IgG1 Fc (N297A) or His tag, (G₃S)₂ linker and AVI tag in the order from the N-terminus to the C-terminus to obtain antigen sequences for immunization and antibody screening and identification (the amino acid sequences were as shown in Table 2, SEQ ID NOs: 7-14). Moreover, General Biosystems (Anhui) Co., Ltd. was commissioned to perform the gene synthesis on the nucleotide sequences corresponding to the amino acid sequences and respectively clone the nucleotide sequences into a signal peptide-containing pTT5 vector, and the plasmids were prepared according to the established standard molecular biology methods. The specific methods could be found in Sambrook, J., Fritsch, E. F., and Maniatis, T. (1989). Molecular Cloning: A Laboratory Manual, Second Edition (Plainview, New York: Cold Spring Harbor Laboratory Press). HEK293E cells (purchased from Suzhou Yiyan Biotechnology Co., Ltd.) were transiently transfected (PEI, Polysciences, catalogue number: 24765-1) and subjected to the scale-up culture using FreeStyle^{™} 293 (Thermofisher scientific, catalogue number: 12338018) at 37°C. After 6 days, the cell culture medium was collected and centrifuged to remove cell components, and the culture supernatant containing the extracellular domain of an antigen protein was obtained and used for purification in the next step.

For the Fc-tagged protein, the culture supernatant was loaded onto a protein A chromatography column (the protein A packing AT Protein A Diamond and the chromatography column BXK16/26 were both purchased from Bestchrom (Shanghai) Biosciences Co., Ltd., with the catalogue numbers AA0273 and B-1620, respectively), and the column was washed with a PBS phosphate buffer (pH 7.4), then washed with 20 mM PB and 1 M NaCl (pH 7.2), and finally eluted with a citric acid buffer (pH 3.4). The Fc-tagged antibody eluted from the protein A chromatography column was collected, neutralized with 1/10 volume of 1 M Tris (pH 8.0), and dialyzed with PBS at 4°C overnight, and the dialyzed protein was aseptically filtered through a 0.22 micron filter membrane and then subpackaged for storage at -80°C to obtain a purified human CD16-Fc protein. The bands of interest of samples detected by SDS-PAGE reducing and non-reducing gels were as shown in FIG. 1.

For the His-tagged antigen, the culture supernatant was loaded onto a nickel ion affinity chromatography column HisTrap^{™} Excel (GE Healthcare, catalogue number: GE17-3712-06), and the change in the ultraviolet absorbance (A280nm) was monitored with an ultraviolet (UV) detector. After sample loading, the nickel ion affinity chromatography column was washed with 20 mM PB and 0.5 M NaCl (pH 7.4) until the ultraviolet absorbance returned to the baseline, and then gradient elutions (2%, 4%, 8%, 16%, 50% and 100%) were performed with buffer A: 20 mM PB and 0.5 M NaCl (pH 7.4), and buffer B: 20 mM PB, 0.5 M NaCl and 500 mM imidazole. The His-tagged human CD16 protein eluted from the nickel ion affinity chromatography column was collected and dialyzed with a PBS phosphate buffer (pH 7.4) overnight in a refrigerator at 4°C. The dialyzed protein was aseptically filtered through a 0.22 micron filter membrane and then subpackaged for storage at-80°C to obtain a purified human CD16-his protein. The bands of interest of samples detected by SDS-PAGE reducing and non-reducing gels were as shown in FIG. 1.

For the preparation of a biotinylated protein, a part of the His-tagged and AVI-tagged CD16 proteins purified in the previous step was taken out for biotin labelling *in vitro.* The biotin labelling was performed according to the operating instructions of a biotin labelling kit (BirA biotin-protein ligase kit, Avidity, catalogue number: BirA500).

**Table 1 Full-length amino acid sequence of CD16 protein**

| **Design ation of sequen ce** | **Sequ ence num ber** | **Amino acid sequence** |
|---|---|---|
| Human CD16a (158F) mutant protein | SEQ ID NO: 1 | MWQLLLPTALLLLVSAGMRTEDLPKAVVFLEPQWYRVLEKDSVTLK CQGAYSPEDNSTQWFHNESLISSQASSYFIDAATVDDSGEYRCQTNLS TLSDPVQLEVHIGWLLLQAPRWVFKEEDPIHLRCHSWKNTALHKVT YLQNGKGRKYFHHNSDFYIPKATLKDSGSYFCRGLFGSKNVSSETVN ITITQGLAVSTISSFFPPGYQVSFCLVMVLLFAVDTGLYFSVKTNIRSST RDWKDHKFKWRKDPQDK |
| Human CD16a (158V) mutant protein | SEQ ID NO: 2 | MWQLLLPTALLLLVSAGMRTEDLPKAVVFLEPQWYRVLEKDSVTLK CQGAYSPEDNSTQWFHNESLISSQASSYFIDAATVDDSGEYRCQTNLS TLSDPVQLEVHIGWLLLQAPRWVFKEEDPIHLRCHSWKNTALHKVT YLQNGKGRKYFHHNSDFYIPKATLKDSGSYFCRGLVGSKNVSSETVN ITITQGLAVSTISSFFPPGYQVSFCLVMVLLFAVDTGLYFSVKTNIRSST RDWKDHKFKWRKDPQDK |
| Human CD16b (NA1) mutant protein | SEQ ID NO: 3 | MWQLLLPTALLLLVSAGMRTEDLPKAVVFLEPQWYRVLEKDSVTLK CQGAYSPEDNSTQWFHNENLISSQASSYFIDAATVDDSGEYRCQTNLS TLSDPVQLEVHVGWLLLQAPRWVFKEEDPIHLRCHSWKNTALHKVT YLQNGKDRKYFHHNSDFHIPKATLKDSGSYFCRGLVGSKNVSSETVN ITITQGLAVSTISSFSPPGYQVSFCLVMVLLFAVDTGLYFSVKTNI |
| Human CD16b (NA2) mutant protein | SEQ ID NO: 4 | MWQLLLPTALLLLVSAGMRTEDLPKAVVFLEPQWYSVLEKDSVTLK CQGAYSPEDNSTQWFHNESLISSQASSYFIDAATVNDSGEYRCQTNLS TLSDPVQLEVHIGWLLLQAPRWVFKEEDPIHLRCHSWKNTALHKVT YLQNGKDRKYFHHNSDFHIPKATLKDSGSYFCRGLVGSKNVSSETVN ITITQGLAVSTISSFSPPGYQVSFCLVMVLLFAVDTGLYFSVKTNI |
| Human CD16b (SH) | SEQ ID NO: | MWQLLLPTALLLLVSAGMRTEDLPKAVVFLEPQWYSVLEKDSVTLK CQGAYSPEDNSTQWFHNESLISSQASSYFIDDATVNDSGEYRCQTNLS TLSDPVQLEVHIGWLLLQAPRWVFKEEDPIHLRCHSWKNTALHKVT |
| mutant protein | 5 | YLQNGKDRKYFHHNSDFHIPKATLKDSGSYFCRGLVGSKNVSSETVN ITITQGLAVSTISSFSPPGYQVSFCLVMVLLFAVDTGLYFSVKTNI |
| Cynom olgus monke y CD16 protein | SEQ ID NO: 6 | MWQLLLPTALLLLVSAGMRAEDLPRAVVFLEPQWYRVLEKDRVTLK CQGAYSPEDNSTRWFHNESLISSQTSSYFIAAARVNNSGEYRCQTSLS TLSDPVQLEVHIGWLLLQAPRWVFKEEESIHLRCHSWKNTLLHKVTY LQNGKGRKYFHQNSDFYIPKATLKDSGSYFCRGLIGSKNVSSETVNIT ITQDLAVSSISSFFPPGYQVSFCLVMVLLFAVDTGLYFSMKKSIPSSTRD WEDHKFKWSKDPQDK |

**Table 2 Antigen amino acid sequence of tagged CD16 extracellular domain**

| **Designat ion of sequence** | **Sequ ence num ber** | **Amino acid sequence** |
|---|---|---|
| Human CD16a (158F)-Fc | SEQ ID NO: 7 | GMRTEDLPKAVVFLEPQWYRVLEKDSVTLKCQGAYSPEDNST QWFHNESLISSQASSYFIDAATVDDSGEYRCQTNLSTLSDPVQL EVHIGWLLLQAPRWVFKEEDPIHLRCHSWKNTALHKVTYLQN GKGRKYFHHNSDFYIPKATLKDSGSYFCRGLFGSKNVSSETVNI TITQGLAVSTISSFFPPGYQ EPKSADKTHTCPPCPAPELLGGPSVFLFPPKPKDTLMISRTPEVTCV VVDVSHEDPEVKFNWYVDGVEVHNAKTKPREEQYASTYRVVSVL TVLHQDWLNGKEYKCKVSNKALPAPIEKTISKAKGQPREPQVYTL PPSREEMTKNQVSLTCLVKGFYPSDIAVEWESNGQPENNYKTTPPV LDSDGSFFLYSKLTVDKSRWQQGNVFSCSVMHEALHNHYTQKSLS LSPGK |
| Human CD16a (158V)-Fc | SEQ ID NO: 8 | GMRTEDLPKAVVFLEPQWYRVLEKDSVTLKCQGAYSPEDNST QWFHNESLISSQASSYFIDAATVDDSGEYRCQTNLSTLSDPVQL EVHIGWLLLQAPRWVFKEEDPIHLRCHSWKNTALHKVTYLQN GKGRKYFHHNSDFYIPKATLKDSGSYFCRGLVGSKNVSSETVNI TITQGLAVSTISSFFPPGYQ EPKSADKTHTCPPCPAPELLGGPSVFLFPPKPKDTLMISRTPEVTCV VVDVSHEDPEVKFNWYVDGVEVHNAKTKPREEQYASTYRVVSVL TVLHQDWLNGKEYKCKVSNKALPAPIEKTISKAKGQPREPQVYTL PPSREEMTKNQVSLTCLVKGFYPSDIAVEWESNGQPENNYKTTPPV |
| | | LDSDGSFFLYSKLTVDKSRWQQGNVFSCSVMHEALHNHYTQKSLS LSPGK |
| Human CD16a (158F)-his | SEQ ID NO: 9 | GMRTEDLPKAVVFLEPQWYRVLEKDSVTLKCQGAYSPEDNST QWFHNESLISSQASSYFIDAATVDDSGEYRCQTNLSTLSDPVQL EVHIGWLLLQAPRWVFKEEDPIHLRCHSWKNTALHKVTYLQN GKGRKYFHHNSDFYIPKATLKDSGSYFCRGLFGSKNVSSETVNI TITQGLAVSTISSFFPPGYQglndifeaqkiewhegggsgggshhhhhhhhhh |
| Human CD16a (158V)-his | SEQ ID NO: 10 | GMRTEDLPKAVVFLEPQWYRVLEKDSVTLKCQGAYSPEDNST QWFHNESLISSQASSYFIDAATVDDSGEYRCQTNLSTLSDPVQL EVHIGWLLLQAPRWVFKEEDPIHLRCHSWKNTALHKVTYLQN GKGRKYFHHNSDFYIPKATLKDSGSYFCRGLVGSKNVSSETVNI TITQGLAVSTISSFFPPGYQglndifeaqkiewheggsgggshhhhhhhhhh |
| Human CD16b (NA1)-his | SEQ ID NO: 11 | GMRTEDLPKAVVFLEPQWYRVLEKDSVTLKCQGAYSPEDNST QWFHNENLISSQASSYFIDAATVDDSGEYRCQTNLSTLSDPVQL EVHVGWLLLQAPRWVFKEEDPIHLRCHSWKNTALHKVT YLQNGKDRKYFHHNSDFHIPKATLKDSGSYFCRGLVGSKNVSS ETVNITITQGLAVSTISgggsgggshhhhhhhhhhglndifeaqkiewhe |
| Human CD16b (NA2)-his | SEQ ID NO: 12 | GMRTEDLPKAVVFLEPQWYSVLEKDSVTLKCQGAYSPEDNSTQ WFHNESLISSQASSYFIDAATVNDSGEYRCQTNLSTLSDPVQLE VHIGWLLLQAPRWVFKEEDPIHLRCHSWKNTALHKVTYLQNG KDRKYFHHNSDFHIPKATLKDSGSYFCRGLVGSKNVSSETVNIT ITQGLAVSTISgggsgggshhhhhhhhhhglndifeaqkiewhe |
| Human CD16b (SH)-his | SEQ ID NO: 13 | GMRTEDLPKAVVFLEPQWYSVLEKDSVTLKCQGAYSPEDNSTQ WFHNESLISSQASSYFIDDATVNDSGEYRCQTNLSTLSDPVQLE VHIGWLLLQAPRWVFKEEDPIHLRCHSWKNTALHKVTYLQNG KDRKYFHHNSDFHIPKATLKDSGSYFCRGLVGSKNVSSETVNIT ITQGLAVSTISgggsgggshhhhhhhhhglndifeaqkiewhe |
| Cynomol gus monkey CD16-his | SEQ ID NO: 14 | GMRAEDLPRAVVFLEPQWYRVLEKDRVTLKCQGAYSPEDNST RWFHNESLISSQTSSYFIAAARVNNSGEYRCQTSLSTLSDPVQLE VHIGWLLLQAPRWVFKEEESIHLRCHSWKNTLLHKVTYLQNG KGRKYFHQNSDFYIPKATLKDSGSYFCRGLIGSKNVSSETVNITI TQDLAVSSISSFFPPGYQglndifeaqkiewhegggsgggshhhhhhhhhh |

| | | |
|---|---|---|
| Notes: the uppercase bold font is the extracellular domain; the underlined uppercase is the Fc tag; GGGSGGGS is the linker; HHHHHHHHHH is the His tag; and the lowercase bold font is the AVI tag. | | |

### 1.2 Preparation of CD16 antibody detected

The mouse antibody 3G8 recognizing human CD16 was obtained by immunizing mice with PMN (Fleit H.B. et al., Proc Natl Acad Sci USA. 1982 May), which could recognize human CD16a (158F and 158V) and CD16b (NA1, NA2 and SH), and had a cross-binding activity with cynomolgus monkey CD16. The VL and VH of 3G8 and P2C47, and the human IgG1 Fc were linked in the order from the N-terminus to the C-terminus, wherein the VL and VH were linked by (GGGGS)₃ linkers to form the form of scFv-hFc, and the corresponding amino acid sequence information was as shown in Table 3 below. The corresponding nucleotide sequences were respectively cloned into a pTT5 vector (completed by General Biosystems (Anhui) Co., Ltd.), and expressed in HEK293E cells (purchased from Suzhou Yiyan Biotechnology Co., Ltd.) and purified according to the method of Example 1.1. NC was the negative control antibody scFv-hFc (L234A, L235A and D265A), which was an antibody against a chicken egg lysozyme, and was custom-made by Taizhou Biointron Biotechnology Co., Ltd.

**Table 3 Sequence information of anti-human CD16 antibody**

| **Designat ion of sequence** | **Sequ ence num ber** | **Amino acid sequence** |
|---|---|---|
| 3G8 light chain variable region | SEQ ID NO: 15 | DTVLTQSPASLAVSLGQRATISCKASQSVDFDGDSFMNWYQQKPGQPP KLLIYTTSNLESGIPARFSASGSGTDFTLNIHPVEEEDTATYYCQQSNEDP YTFGGGTKLEIK |
| 3G8 heavy chain variable region | SEQ ID NO: 16 | QVTLKESGPGILQPSQTLSLTCSFSGFSLRTSGMGVGWIRQPSGKGLEW LAHIWWDDDKRYNPALKSRLTISKDTSSNQVFLKIASVDTADTATYYC AQINPAWFAYWGQGTLVTVSA |
| 3G8 scFv-hFc | SEQ ID NO: 17 | DTVLTQSPASLAVSLGQRATISCKASQSVDFDGDSFMNWYQQKPGQPP KLLIYTTSNLESGIPARFSASGSGTDFTLNIHPVEEEDTATYYCQQSNEDP YTFGGGTKLEIKGGGGSGGGGSGGGGSQVTLKESGPGILQPSQTLSLTC SFSGFSLRTSGMGVGWIRQPSGKGLEWLAHIWWDDDKRYNPALKSRL TISKDTSSNQVFLKIASVDTADTATYYCAQINPAWFAYWGQGTLVTVSA EPKSSDKTHTCPPCPAPEEFGGPSVFLFPPKPKDTLMISRTPEVTCV VVAVSHEDPEVKFNWYVDGVEVHNAKTKPREEQYNSTYRVVSVLT VLHQDWLNGKEYKCKVSNKALPAPIEKTISKAKGQPREPQVYTLP PSREEMTKNQVSLTCLVKGFYPSDIAVEWESNGQPENNYKTTPPVL |
| | | DSDGSFFLYSKLTVDKSRWQQGNVFSCSVMHEALHNHYTQKSLSL SPGK |
| P2C47 heavy chain variable region | SEQ ID NO: 18 | QVQLVQSGAEVKKPGESLKVSCKASGYTFTSYYMHWVRQAPGQGLE WMGAIEPMYGSTSYAQKFQGRVTMTRDTSTSTVYMELSSLRSEDTAVY YCARGSAYYYDFADYWGQGTLVTVSS |
| P2C47 light chain variable region | SEQ ID NO: 19 | SYVLTQPSSVSVAPGQTATISCGGHNIGSKNVHWYQQRPGQSPVLVIYQ DNKRPSGIPERFSGSNSGNTATLTISGTQAMDEADYYCQVWDNYSVLF GGGTKLTVL |
| P2C47 scFv-hFc | SEQ ID NO: 20 | SYVLTQPSSVSVAPGQTATISCGGHNIGSKNVHWYQQRPGQSPVLVIYQ DNKRPSGIPERFSGSNSGNTATLTISGTQAMDEADYYCQVWDNYSVLF GGGTKLTVLGGGGSGGGGSGGGGSQVQLVQSGAEVKKPGESLKVSCK ASGYTFTSYYMHWVRQAPGQGLEWMGAIEPMYGSTSYAQKFQGRVT MTRDTSTSTVYMELSSLRSEDTAVYYCARGSAYYYDFADYWGQGTLV TVSSEPKSSDKTHTCPPCPAPEEFGGPSVFLFPPKPKDTLMISRTPEV TCVVVAVSHEDPEVKFNWYVDGVEVHNAKTKPREEQYNSTYRVVS VLTVLHQDWLNGKEYKCKVSNKALPAPIEKTISKAKGQPREPQVY TLPPSREEMTKNQVSLTCLVKGFYPSDIAVEWESNGQPENNYKTTP PVLDSDGSFFLYSKLTVDKSRWQQGNVFSCSVMHEALHNHYTQKS LSLSPGK |

| | | |
|---|---|---|
| Notes: the bold font is the human hIgG1 Fc sequence. | | |

### 1.3 Construction of Flipin CHO cell line stably transfected with CD16

The nucleotide sequences encoding the full-length amino acid sequence of human CD16a158F (Uniprot ID: P08637, SEQ ID NO: 1), the full-length amino acid sequence of human CD16a158V (NCBI ID: AAH17865.1, SEQ ID NO: 2), the full-length amino acid sequence of human CD16b (NA1) (NCBI ID: AAA35881.1, SEQ ID NO: 3), the full-length amino acid sequence of human CD16b (NA2) (Uniprot ID: 075015, SEQ ID NO: 4), and the full-length amino acid sequence of cynomolgus monkey CD16 (NCBI ID: NP_001270121.1, SEQ ID NO: 6) were cloned into a pcDNA5-FRT vector (Thermofisher scientific, catalogue number: V601020), respectively, and the plasmids were prepared (completed by Sangon Bioengineering (Shanghai) Co., Ltd.). The prepared plasmids were respectively co-transfected with a POG44 enzyme vector (Thermofisher scientific, catalogue number: V600520) into a FlpinCHO cell line (Thermofisher scientific, catalogue number: R75807), and the transfection method was carried out according to the operating instructions of a transfection reagent (Lipofectamine^{®} 3000 Transfection Kit, Invitrogen, catalogue number: L3000-015). The transfected FlipinCHO cells were placed in an incubator at 37°C and 5% (v/v) CO₂, and selectively cultured in an F12 medium containing 800 µg/ml hygromycin and 10% (w/w) foetal bovine serum. About 2 weeks later, some cells were taken to detect the expression of antigens on the cell surface by flow cytometry, and the recovered cell lines were subjected to the further scale-up culture and frozen in liquid nitrogen.

As shown in Table 4 and FIG. 2, the IgG isotype control was a murine IgG1 control (purchased from Taizhou Biointron Biotechnology Co., Ltd., catalogue number: B118301). As shown in FIG. 2, the 3G8 antibody could bind to Flpin CHO-human CD16a (158F), Flpin CHO-human CD16a (158V), Flpin CHO-human CD16b (NA1), Flpin CHO-human CD16b (NA2) and Flpin CHO-cyno CD16 cell lines. The mean fluorescence intensities of the transfected cell lines stained with the 3G8 antibody and a fluorescent secondary antibody were listed in Table 4, indicating that these cell lines had been able to stably express the corresponding CD16 antigen and could be used for the subsequent antibody screening and identification.

**Table 4 FACS detection results of Flpin CHO cell line stably transfected with CD16 full-length protein**

| No. | Designation of stably transfected cell line | Mean fluorescence intensity | |
|---|---|---|---|
| | | IgG isotype control | CD16 antibody |
| 1 | FlpinCHO-human CD16a (158F) | 67 | 3075 |
| 2 | FlpinCHO-human CD16a (158V) | 79 | 5868 |
| 3 | FlpinCHO-human CD16b (NA1) | 81 | 28093 |
| 4 | FlpinCHO-human CD16b (NA2) | 76 | 24764 |
| 5 | FlpinCHO-cyno CD16 | 89 | 5503 |

### Example 2 Screening of single domain antibody against human CD16

### 2.1 Immunization and serum titre detection of llama

Human CD16a (158F)-hFc and human CD16a (158V)-hFc proteins were used for immunization. One llama was selected for immunization four times with an interval of 3 weeks. After the third and fourth immunizations, peripheral blood was collected and serum was separated, and enzyme-linked immunosorbent assay (ELISA) was used to detect the antibody titre and specificity against human CD16 in serum. The results were as shown in Table 5. Table 5 indicated that the serum of the llama immunized with human CD16 binded to the immunogen to varying degrees, showing an antigen-antibody reaction, and the highest dilution was about 5.9 million. Specifically, the blank control is 1% (w/w) BSA, the batch refers to the llama serum on the seventh day after the third (TB3) and fourth (TB4) immunizations, and the data in the table are OD450 nm values.

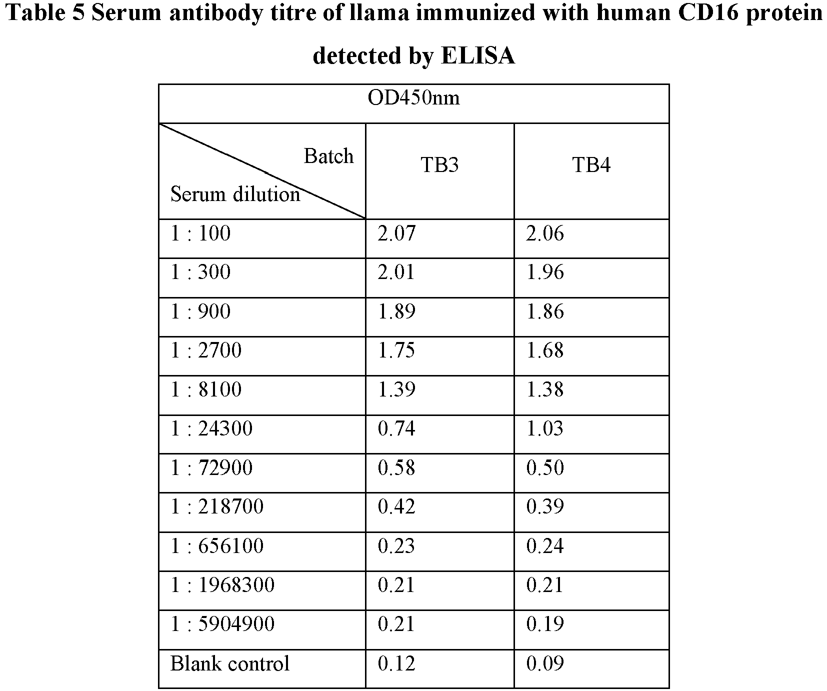

### 2.2 Library construction

A total of 100 mL of llama peripheral blood was collected after three immunizations and after four immunizations. Lymphocyte separation medium was used to isolate PBMC, RNAiso Plus reagent (Takara, catalogue number: #9108/9109) was used to extract total RNA, and PrimeScript^{™} II 1st Strand cDNA Synthesis Kit (Takara, catalogue number: 6210A) was used to reverse transcribe the extracted RNA into cDNA. Nested PCR was used to amplify the variable region nucleic acid fragment encoding the nanobody and the nucleic acid fragment of the nanobody of interest was recovered. The nucleic acid fragment of the nanobody and the linearized yeast display vector pYDC2 (from Sichuan NB Biolab Co., Ltd.) were electrotransformed into yeast competent cell EYB100 for *in vivo* recombination, and a nanobody yeast display library against CD16 was constructed and tested. By gradient dilution plating, the calculated library size was 3.26 × 10⁸. To test the insertion rate of the library, 48 clones were randomly selected for colony PCR. The results showed that the insertion rate reached 100%.

### 2.3 Panning of nanobody VHH against CD16

The yeast display library was reverse-screened after the magnetic beads were coated with the biotinylated human CD16b(NA1)-his, CD16b (NA2)-his and CD16b (SH)-his proteins prepared in Example 1.1, and then forward-screened after the magnetic beads were coated with the biotinylated human CD16a (158F)-his and CD16a (158V)-his proteins prepared in Example 1.1. After the yeast clones specifically binding to human CD16a were enriched, flow cytometry was used to perform the reverse screening of the fluorescein-labelled human CD16b(NA1)-his, CD16b (NA2)-his and CD16b (SH)-his proteins and the forward screening of human CD16a (158F)-his and CD16a (158V)-his proteins to obtain the yeast clone specifically binding to CD16a.

### 2.4 Screening of single specific positive clone by flow cytometry

After panning, the obtained CD16a (158F) and CD16a (158V)-binding positive yeasts were plated and amplified, single clones were selected, and then 96 single colonies were selected for expansion and culture separately. Biotinylated CD16a (158F)-his protein was incubated with excess SA-iFluor 647 (purchased from ThermoFisher scientific, catalogue number: S21374), biotinylated CD16b (NA1)-his, CD16b (NA2)-his, and CD16b (SH)-his proteins with excess SA-Alexa Fluor 488 (purchased from ThermoFisher scientific, catalogue number: S11223) separately for 1 hour for fluorescence labelling until use. The expressed yeast cells were washed and then divided into two parts. A part of the yeast cells were incubated with a human immunoglobulin (Shanghai Institute of Biological Products Co., Ltd.) and the aforementioned fluorescein-labelled CD16a and CD16b antigens at 4°C for 1 hour; the other part of the yeast cells were incubated with Alexa Fluor 488-labelled anti-HA antibody (purchased from ThermoFisher scientific, catalogue number: A-21287) at 4°C for 1 hour. After the yeast cells were washed, a flow cytometer was used for analysis, and the mean fluorescence intensities (MFIs) were calculated, respectively. The MFIs of the fluorescently labelled CD16a and CD16b were divided by the MFI of the fluorescently labelled anti-HA, respectively, to obtain the normalized MFI values of CD16a and CD16b. The normalized MFI values of some clones were as shown in Table 6.

**Table 6 Normalized fluorescence intensity value of CD16a and CD16b of monoclonal yeast cell**

| Clone | Normalized MFI | |
|---|---|---|
| | CD 16a | CD16b |
| A07 | 4524.36 | 114.80 |
| A09 | 3952.99 | 73.96 |
| A11 | 2068.61 | 74.63 |
| A12 | 2831.17 | 99.67 |
| B07 | 4314.55 | 117.75 |
| B08 | 2811.46 | 65.74 |
| B10 | 2566.53 | 91.14 |
| B12 | 4103.03 | 121.82 |
| C08 | 3658.52 | 77.47 |
| C09 | 2336.02 | 60.39 |
| C10 | 3121.39 | 72.25 |
| C11 | 3407.26 | 99.65 |
| C12 | 2821.22 | 60.85 |
| D10 | 2415.61 | 70.52 |
| D12 | 2161.58 | 71.33 |
| E07 | 3048.26 | 71.69 |
| E08 | 2575.77 | 83.50 |
| E09 | 2465.28 | 63.77 |
| E10 | 2335.62 | 64.38 |
| E11 | 2549.77 | 78.30 |

CD16a-positive clones were selected for sequencing. The sequencing results were analysed using MOE software, and the phylogenetic tree was constructed according to the amino acid sequence of the VHH coding protein. After the sequences that were close to each other on the phylogenetic tree were eliminated according to the sequence similarity, 14 clones were obtained by screening. The nanobody sequences were as shown in Table 7. Table 8 showed the amino acid sequences of fusions of VHH and Fc segments. The CDRs of the antibody sequences were analysed using KABAT, Chothia or IMGT software, respectively, and the CDR sequence information was as shown in Table 9 below. Subsequently, the production and identification of the VHH nanobody-Fc fusion protein were carried out according to the method in Example 1.2.

**Table 7 Amino acid sequence of VHH antibody**

| **Designation of sequence** | **Sequence number** | **Amino acid sequence** |
|---|---|---|
| VHH01 | SEQ ID NO: 21 | QVQLVESGGGLVQAGGSLRLSCVASRSTLTTYAVRWYRQAPGKQREWV AFISVDGNTNYPDSVKGRFIVSRDNAKNTVYLQMNSLKPEDTADYICRA GGQYWGQGTQVTVSS |
| VHH02 | SEQ ID NO: 22 | EVQLVESGGGLVQAGGSLRLSCATSGITFSSYAMGWYRQAPGKQRELVA GINRFGLTNYADSVKGRFTISRDNAKNTVYLQMTSLKSEDTAVYYCNTG GVYSHPNYYTGIYWGQGTQVTVSS |
| VHH04 | SEQ ID NO: 23 | QVQLVESGGGLVQAGGSLRLSCVGSSSTLTTYAVRWYRQAPGKQREWV AFISVDGNTNYPDSVKGRFIVSRDNAKNTVYLQMNNLKPEDTADYICRA GGQYWGQGTQVTVSS |
| VHH05 | SEQ ID NO: 24 | QVQLVESGGGLVQAGGSLRLSCVASSSTVTTYAVRWYRQAPGKQREWV AFISVDGNTNYPDSVKGRFIVSRDNAKNTVYLQMNSLKPEDTADYICRA GGQYWGQGTQVTVSS |
| VHH06 | SEQ ID NO: 25 | EVQLVESGGGLVQAGGSLRLSCVASGSTWTTYAVRWYRQAPGKQREWV AFISVDGSTNYPDSVKGRFIVSRDNAKNTVYLQMNSLKPEDTADYICRV GGQYWGQGTQVTVSS |
| VHH07 | SEQ ID NO: 26 | EVQLVESGGGLVQAGGSLRLSCVASRSTLTTYAVRWYRQAPGKQREWVA FISVDGKTNYPDSVKGRFIVSRDNAKNTVYLQMNSLKPEDTADYICRAG GQYWGQGTQVTVSS |
| VHH08 | SEQ ID NO: 27 | EVQVVESGGGLVQAGGSLRLSCVASRSTLSTYAVRWYRQAPGNQREWV AFISVDGNTNYPDSVKGRFIVSRDNAKNTVYLQMNSLKPEDTADYICRA GGQYWGQGTQVTVSS |
| VHH09 | SEQ ID NO: 28 | EVQVVESGGGLVQAGGSLRLSCVASTSTLTTYAVRWYRQAPGKQREWV AFISVDGNTNYPDSVKGRFIVSRDNAKNTVYLQMNSLKPEDTADYICRA GGQYWGQGTQVTVSS |
| VHH10 | SEQ ID NO: 29 | QVQLVESGGGLVQAGGSLRLSCVGSSSTLTTYAVRWYRQAPGKQREWV AFISVDGSTNYPDSVKGRFIVSRDNAKNTVYLQMNSLKPEDTADYICRA GGQYWGQGTQVTVSS |
| VHH11 | SEQ ID NO: 30 | EVQVVESGGGLAQAGGSLRLSCAVSGITFSSYAMGWYRQDPGKQRELVA AINRFGLTNYADSVKGRFTISRDNAKNTVYLQMTSLKPEDTAVYYCNTG GVYSHPNYYTGTYWGQGTQVTVSS |
| VHH12 | SEQ ID NO: 31 | QVQLVESGGGLVQAGGSLRLSCAASGITFSSYAMGWYRQAPGKQRELVA AINRFGLTNYADFVKGRFTISRVNAKNTVYLQMTSLKPEDTAVYYCNTG GVYSHPDYYTGTYWGQGTQVTVSS |
| VHH13 | SEQ ID NO: 32 | EVQVVESGGGLVQAGGSLRLSCAASGITFSSYAMGWYRQAPGKQRELVA AINRFGLTNYADSVKGRFTISRDNAKSTVYLQMTSLKPEDTAVYYCNTG GVYSHPDYYTGTYWGQGTQVTVSS |
| VHH14 | SEQ ID NO: 33 | EVQLVESGGGLVQAGGSLRLSCAASGITFSSYAMAWYRQDPGKQRELVA AINRFGLTNYADSVKGRFTISRDNAKNTVYLEMTSLKPEDTAVYYCNTG GVYSHPNYYTGTYWGQGTQVTVSS |
| VHH15 | SEQ ID NO: 34 | EVQLVESGGGLVQAGGSLRLSCVASTSTLTTYAVRWYRQAPGKQREWVA FISVDGNTNYPDSVKGRFIVSRDNAKNTVYLQMNSLKPEDTADYICRAG GQYWGQGTQVTVSS |

**Table 8 Amino acid sequence of VHH-Fc antibody**

| **Design ation of sequen ce** | **Sequ ence num ber** | **Amino acid sequence** |
|---|---|---|
| VHH01 | SEQ ID NO: 35 | QVQLVESGGGLVQAGGSLRLSCVASRSTLTTYAVRWYRQAPGKQRE WVAFISVDGNTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH02 | SEQ ID NO: 36 | EVQLVESGGGLVQAGGSLRLSCATSGITFSSYAMGWYRQAPGKQRE LVAGINRFGLTNYADSVKGRFTISRDNAKNTVYLQMTSLKSEDTAVY YCNTGGVYSHPNYYTGIYWGQGTQVTVSSEPKSSDKTHTCPPCPA PEFEGGPSVFLFPPKPKDTLMISRTPEVTCVVVAVSHEDPEVKFN WYVDGVEVHNAKTKPREEQYNSTYRVVSVLTVLHQDWLNGKE YKCKVSNKALPAPIEKTISKAKGQPREPQVYTLPPSREEMTKNQ VSLTCLVKGFYPSDIAVEWESNGQPENNYKTTPPVLDSDGSFFLY SKLTVDKSRWQQGNVFSCSVMHEALHNHYTQKSLSLSPGK |
| VHH04 | SEQ ID NO: 37 | QVQLVESGGGLVQAGGSLRLSCVGSSSTLTTYAVRWYRQAPGKQRE WVAFISVDGNTNYPDSVKGRFIVSRDNAKNTVYLQMNNLKPEDTA DYICRAGGQYWGQGTQVTVSSEPKSSDKTHTCPPCPAPEFEGGPS VFLFPPKPKDTLMISRTPEVTCVVVAVSHEDPEVKFNWYVDGVE VHNAKTKPREEQYNSTYRVVSVLTVLHQDWLNGKEYKCKVSN KALPAPIEKTISKAKGQPREPQVYTLPPSREEMTKNQVSLTCLVK GFYPSDIAVEWESNGQPENNYKTTPPVLDSDGSFFLYSKLTVDKS RWQQGNVFSCSVMHEALHNHYTQKSLSLSPGK |
| VHH05 | SEQ ID NO: | QVQLVESGGGLVQAGGSLRLSCVASSSTVTTYAVRWYRQAPGKQRE WVAFISVDGNTNYPDSVKGRFIVSRDNAKNTVYLQMNSLKPEDTAD |
| | 38 | YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH06 | SEQ ID NO: 39 | EVQLVESGGGLVQAGGSLRLSCVASGSTWTTYAVRWYRQAPGKQR EWVAFISVDGSTNYPDSVKGRFIVSRDNAKNTVYLQMNSLKPEDTA DYICRVGGQYWGQGTQVTVSSEPKSSDKTHTCPPCPAPEFEGGPS VFLFPPKPKDTLMISRTPEVTCVVVAVSHEDPEVKFNWYVDGVE VHNAKTKPREEQYNSTYRVVSVLTVLHQDWLNGKEYKCKVSN KALPAPIEKTISKAKGQPREPQVYTLPPSREEMTKNQVSLTCLVK GFYPSDIAVEWESNGQPENNYKTTPPVLDSDGSFFLYSKLTVDKS RWQQGNVFSCSVMHEALHNHYTQKSLSLSPGK |
| VHH07 | SEQ ID NO: 40 | EVQLVESGGGLVQAGGSLRLSCVASRSTLTTYAVRWYRQAPGKQRE WVAFISVDGKTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH08 | SEQ ID NO: 41 | EVQVVESGGGLVQAGGSLRLSCVASRSTLSTYAVRWYRQAPGNQRE WVAFISVDGNTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH09 | SEQ ID NO: 42 | EVQVVESGGGLVQAGGSLRLSCVASTSTLTTYAVRWYRQAPGKQRE WVAFISVDGNTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH10 | SEQ ID NO: 43 | QVQLVESGGGLVQAGGSLRLSCVGSSSTLTTYAVRWYRQAPGKQRE WVAFISVDGSTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH11 | SEQ ID NO: 44 | EVQVVESGGGLAQAGGSLRLSCAVSGITFSSYAMGWYRQDPGKQR ELVAAINRFGLTNYADSVKGRFTISRDNAKNTVYLQMTSLKPEDTAV YYCNTGGVYSHPNYYTGTYWGQGTQVTVSSEPKSSDKTHTCPPCP APEFEGGPSVFLFPPKPKDTLMISRTPEVTCVVVAVSHEDPEVKF NWYVDGVEVHNAKTKPREEQYNSTYRVVSVLTVLHQDWLNGK EYKCKVSNKALPAPIEKTISKAKGQPREPQVYTLPPSREEMTKN QVSLTCLVKGFYPSDIAVEWESNGQPENNYKTTPPVLDSDGSFFL YSKLTVDKSRWQQGNVFSCSVMHEALHNHYTQKSLSLSPGK |
| VHH12 | SEQ ID NO: 45 | QVQLVESGGGLVQAGGSLRLSCAASGITFSSYAMGWYRQAPGKQRE LVAAINRFGLTNYADFVKGRFTISRVNAKNTVYLQMTSLKPEDTAVY YCNTGGVYSHPDYYTGTYWGQGTQVTVSSEPKSSDKTHTCPPCPA PEFEGGPSVFLFPPKPKDTLMISRTPEVTCVVVAVSHEDPEVKFN WYVDGVEVHNAKTKPREEQYNSTYRVVSVLTVLHQDWLNGKE YKCKVSNKALPAPIEKTISKAKGQPREPQVYTLPPSREEMTKNQ VSLTCLVKGFYPSDIAVEWESNGQPENNYKTTPPVLDSDGSFFLY SKLTVDKSRWQQGNVFSCSVMHEALHNHYTQKSLSLSPGK |
| VHH13 | SEQ ID NO: 46 | EVQVVESGGGLVQAGGSLRLSCAASGITFSSYAMGWYRQAPGKQR ELVAAINRFGLTNYADSVKGRFTISRDNAKSTVYLQMTSLKPEDTAV YYCNTGGVYSHPDYYTGTYWGQGTQVTVSSEPKSSDKTHTCPPCP APEFEGGPSVFLFPPKPKDTLMISRTPEVTCVVVAVSHEDPEVKF NWYVDGVEVHNAKTKPREEQYNSTYRVVSVLTVLHQDWLNGK EYKCKVSNKALPAPIEKTISKAKGQPREPQVYTLPPSREEMTKN QVSLTCLVKGFYPSDIAVEWESNGQPENNYKTTPPVLDSDGSFFL YSKLTVDKSRWQQGNVFSCSVMHEALHNHYTQKSLSLSPGK |
| VHH14 | SEQ ID NO: 47 | EVQLVESGGGLVQAGGSLRLSCAASGITFSSYAMAWYRQDPGKQRE LVAAINRFGLTNYADSVKGRFTISRDNAKNTVYLEMTSLKPEDTAVY YCNTGGVYSHPNYYTGTYWGQGTQVTVSSEPKSSDKTHTCPPCPA PEFEGGPSVFLFPPKPKDTLMISRTPEVTCVVVAVSHEDPEVKFN WYVDGVEVHNAKTKPREEQYNSTYRVVSVLTVLHQDWLNGKE YKCKVSNKALPAPIEKTISKAKGQPREPQVYTLPPSREEMTKNQ |
| | | VSLTCLVKGFYPSDIAVEWESNGQPENNYKTTPPVLDSDGSFFLY SKLTVDKSRWQQGNVFSCSVMHEALHNHYTQKSLSLSPGK |
| VHH15 | SEQ ID NO: 48 | EVQLVESGGGLVQAGGSLRLSCVASTSTLTTYAVRWYRQAPGKQRE WVAFISVDGNTNYPDSVKGRFIVSRDNAKNTVYLQMNSLKPEDTAD YICRAGGQYWGQGTQVTVSSEPKSSDKTHTCPPCPAPEFEGGPSV FLFPPKPKDTLMISRTPEVTCVVVAVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| hIgGIF c | SEQ ID NO: 89 | EPKSSDKTHTCPPCPAPEFEGGPSVFLFPPKPKDTLMISRTPEVT CVVVAVSHEDPEVKFNWYVDGVEVHNAKTKPREEQYNSTYRVV SVLTVLHQDWLNGKEYKCKVSNKALPAPIEKTISKAKGQPREPQ VYTLPPSREEMTKNQVSLTCLVKGFYPSDIAVEWESNGQPENNY KTTPPVLDSDGSFFLYSKLTVDKSRWQQGNVFSCSVMHEALHN HYTQKSLSLSPGK |

| | | |
|---|---|---|
| Notes: the bold font is the human hIgGlFc sequence. | | |

**Table 9 CDR sequence information**

| **Designat ion of antibody** | **Analysis method** | **CDR1** | **CDR2** | **CDR3** |
|---|---|---|---|---|
| VHH0 1 | Kabat | TYAVR (SEQ ID NO: 49) | FISVDGNTNYPDSVKG (SEQ ID NO: 50) | GGQY (SEQ ID NO: 51) |
| | Chothia | RSTLTTY (SEQ ID NO: 52) | SVDGN (SEQ ID NO: 53) | GGQY (SEQ ID NO: 51) |
| | IMGT | RSTLTTYA (SEQ ID NO: 54) | ISVDGNT (SEQ ID NO: 55) | RAGGQY (SEQ ID NO: 56) |
| VHH02 | Kabat | SYAMG (SEQ ID NO: 57) | GINRFGLTNYADSVKG (SEQ ID NO: 58) | GGVYSHPNYYTGIY (SEQ ID NO: 59) |
| | Chothia | GITFSSY (SEQ ID NO: 60) | NRFGL (SEQ ID NO: 61) | GGVYSHPNYYTGIY (SEQ ID NO: 59) |
| | IMGT | GITFSSYA (SEQ ID NO: 62) | INRFGLT (SEQ ID NO: 63) | NTGGVYSHPNYYTGIY (SEQ ID NO: 64) |
| VHH04 | Kabat | TYAVR (SEQ ID NO: 49) | FISVDGNTNYPDSVKG (SEQ ID NO: 50) | GGQY (SEQ ID NO: 51) |
| | Chothia | SSTLTTY (SEQ ID NO: 65) | SVDGN (SEQ ID NO: 53) | GGQY (SEQ ID NO: 51) |
| | IMGT | SSTLTTYA (SEQ ID NO: 66) | ISVDGNT (SEQ ID NO: 55) | RAGGQY (SEQ ID NO: 56) |
| VHH05 | Kabat | TYAVR (SEQ ID NO: 49) | FISVDGNTNYPDSVKG (SEQ ID NO: 50) | GGQY (SEQ ID NO: 51) |
| | Chothia | SSTVTTY (SEQ ID NO: 67) | SVDGN (SEQ ID NO: 53) | GGQY (SEQ ID NO: 51) |
| | IMGT | SSTVTTYA (SEQ ID NO: 68) | ISVDGNT (SEQ ID NO: 55) | RAGGQY (SEQ ID NO: 56) |
| VHH06 | Kabat | TYAVR (SEQ ID NO: 49) | FISVDGSTNYPDSVKG (SEQ ID NO: 69) | GGQY (SEQ ID NO: 51) |
| | Chothia | GSTWTTY (SEQ ID NO: 70) | SVDGS (SEQ ID NO: 71) | GGQY (SEQ ID NO: 51) |
| | IMGT | GSTWTTYA (SEQ ID NO: 72) | ISVDGST (SEQ ID NO: 73) | RVGGQY (SEQ ID NO: 74) |
| VHH07 | Kabat | TYAVR (SEQ ID NO: 49) | FISVDGKTNYPDSVKG (SEQ ID NO: 75) | GGQY (SEQ ID NO: 51) |
| | Chothia | RSTLTTY (SEQ ID NO: 52) | SVDGK (SEQ ID NO: 76) | GGQY (SEQ ID NO: 51) |
| | IMGT | RSTLTTYA (SEQ ID NO: 54) | ISVDGKT (SEQ ID NO: 77) | RAGGQY (SEQ ID NO: 56) |
| VHH08 | Kabat | TYAVR (SEQ ID NO: 49) | FISVDGNTNYPDSVKG (SEQ ID NO: 50) | GGQY (SEQ ID NO: 51) |
| | Chothia | RSTLSTY (SEQ ID NO: 78) | SVDGN (SEQ ID NO: 53) | GGQY (SEQ ID NO: 51) |
| | IMGT | RSTLSTYA (SEQ ID NO: 79) | ISVDGNT (SEQ ID NO: 55) | RAGGQY (SEQ ID NO: 56) |
| VHH09 | Kabat | TYAVR (SEQ ID NO: 49) | FISVDGNTNYPDSVKG (SEQ ID NO: 50) | GGQY (SEQ ID NO: 51) |
| | Chothia | TSTLTTY (SEQ ID NO: 80) | SVDGN (SEQ ID NO: 53) | GGQY (SEQ ID NO: 51) |
| | IMGT | TSTLTTYA (SEQ ID NO: 81) | ISVDGNT (SEQ ID NO: 55) | RAGGQY (SEQ ID NO: 56) |
| VHH10 | Kabat | TYAVR (SEQ ID NO: 49) | FISVDGSTNYPDSVKG (SEQ ID NO: 69) | GGQY (SEQ ID NO: 51) |
| | Chothia | SSTLTTY (SEQ ID NO: 65) | SVDGS (SEQ ID NO: 71) | GGQY (SEQ ID NO: 51) |
| | IMGT | SSTLTTYA (SEQ ID NO: 66) | ISVDGST (SEQ ID NO: 73) | RAGGQY (SEQ ID NO: 56) |
| VHH11 | Kabat | SYAMG (SEQ ID NO: 57) | AINRFGLTNYADSVKG (SEQ ID NO: 82) | GGVYSHPNYYTGTY (SEQ ID NO: 83) |
| | Chothia | GITFSSY (SEQ ID NO: 60) | NRFGL (SEQ ID NO: 61) | GGVYSHPNYYTGTY (SEQ ID NO: 83) |
| | IMGT | GITFSSYA (SEQ ID NO: 62) | INRFGLT (SEQ ID NO: 63) | NTGGVYSHPNYYTGTY (SEQ ID NO: 84) |
| VHH12 | Kabat | SYAMG | AINRFGLTNYADFVKG | GGVYSHPDYYTGTY |
| | | (SEQ ID NO: 57) | (SEQ ID NO: 85) | (SEQ ID NO: 86) |
| | Chothia | GITFSSY (SEQ ID NO: 60) | NRFGL (SEQ ID NO: 61) | GGVYSHPDYYTGTY (SEQ ID NO: 86) |
| | IMGT | GITFSSYA (SEQ ID NO: 62) | INRFGLT (SEQ ID NO: 63) | NTGGVYSHPDYYTGTY (SEQ ID NO: 87) |
| VHH13 | Kabat | SYAMG (SEQ ID NO: 57) | AINRFGLTNYADSVKG (SEQ ID NO: 82) | GGVYSHPDYYTGTY (SEQ ID NO: 86) |
| | Chothia | GITFSSY (SEQ ID NO: 60) | NRFGL (SEQ ID NO: 61) | GGVYSHPDYYTGTY (SEQ ID NO: 86) |
| | IMGT | GITFSSYA (SEQ ID NO: 62) | INRFGLT (SEQ ID NO: 63) | NTGGVYSHPDYYTGTY (SEQ ID NO: 87) |
| VHH14 | Kabat | SYAMA (SEQ ID NO: 88) | AINRFGLTNYADSVKG (SEQ ID NO: 82) | GGVYSHPNYYTGTY (SEQ ID NO: 83) |
| | Chothia | GITFSSY (SEQ ID NO: 60) | NRFGL (SEQ ID NO: 61) | GGVYSHPNYYTGTY (SEQ ID NO: 83) |
| | IMGT | GITFSSYA (SEQ ID NO: 62) | INRFGLT (SEQ ID NO: 63) | NTGGVYSHPNYYTGTY (SEQ ID NO: 84) |
| VHH15 | Kabat | TYAVR (SEQ ID NO: 49) | FISVDGNTNYPDSVKG (SEQ ID NO: 50) | GGQY (SEQ ID NO: 51) |
| | Chothia | TSTLTTY (SEQ ID NO: 80) | SVDGN (SEQ ID NO: 53) | GGQY (SEQ ID NO: 51) |
| | IMGT | TSTLTTYA (SEQ ID NO: 81) | ISVDGNT (SEQ ID NO: 55) | RAGGQY (SEQ ID NO: 56) |

### Example 3 Detection of binding activity of CD16 antibody

### 3.1 Binding of human CD16 antibody to cells expressing different mutants of CD16a detected by flow cytometry (fluorescence activated cell sorting, FACS)

The required cells were scale-up cultured in a T-75 cell culture flask to the logarithmic growth phase. The medium was aspirated, and the cells were washed twice with a PBS buffer and then digested with trypsin. After the digestion was terminated, the cells were washed twice with the PBS buffer. The cells were counted, and then the cell pellet was resuspended with a [PBS + 2% (w/w) BSA] blocking solution to 2 × 10⁶ cells/ml, and added to a 96-well FACS reaction plate at 50 µl/well. An antibody to be detected had an initial concentration of 100 nM, and was subjected to gradient dilution with PBS at a ratio of 1 : 5. 50 µl/well of the antibody to be detected was added at different concentrations, and incubated on ice for 1 hour. The mixture was centrifuged and washed 3 times with a PBS buffer. iFluor 647-labelled anti-human IgG secondary antibody (purchased from Jackson immune, catalogue number: 109-605-088) was added at 50 µl/well, and incubated on ice for 1 hour. The mixture was centrifuged and washed 5 times with PBS, and FACS (FACS Canto II, purchased from BD Company) was used for detection and result analysis. Data analysis was performed by software (CellQuest) to obtain the mean fluorescence intensity (MFI) of the cells. Then, software (GraphPad Prism8) was used for analysis, data fitting and EC50 value calculation.

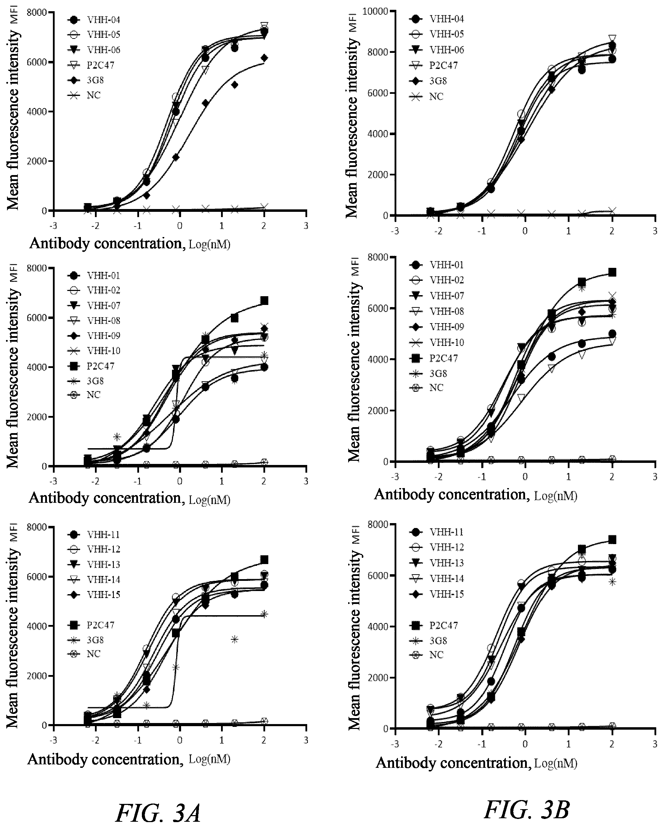

The results were as shown in Table 10 and FIGS. 3A-3B. The CD16 VHH-hFc antibodies had good binding activity to both FlpinCHO-CD16a (158F) and FlpinCHO-CD16a (158V) cells, and had comparable EC50 in terms of binding to the two cells. The EC50 of most antibodies was less than 1 nM.

**Table 10 Binding activity of VHH-Fc antibody with FlpinCHO-CD16a cell detected by FACS**

| Designation of antibody | FlpinCHO-human CD16a (158F) | | FlpinCHO-human CD16a (158V) | |
|---|---|---|---|---|
| | Maximum mean fluorescence intensity | EC50 (nM) | Maximum mean fluorescence intensity | EC50 (nM) |
| VHH-01 | 4009 | 0.91 | 5001 | 0.47 |
| VHH-02 | 5210 | 1.17 | 5997 | 0.30 |
| VHH-07 | 5190 | 0.25 | 5966 | 0.32 |
| VHH-08 | 4245 | 0.56 | 4706 | 0.81 |
| VHH-09 | 5553 | 0.47 | 6243 | 0.69 |
| VHH-10 | 5622 | 0.44 | 6475 | 0.63 |
| VHH-11 | 5672 | 0.28 | 6241 | 0.33 |
| VHH-12 | 6094 | 0.16 | 6679 | 0.21 |
| VHH-13 | 6019 | 0.18 | 6661 | 0.24 |
| VHH-14 | 5754 | 0.23 | 6248 | 0.27 |
| VHH-15 | 5605 | 0.44 | 6489 | 0.69 |
| P2C47 | 6685 | 0.62 | 7408 | 0.85 |
| 3G8 | 4489 | about 0.82 | 6812 | 0.56 |
| NC | 162 | Negative | 97 | Negative |
| VHH-04 | 7235 | 0.66 | 7658 | 0.68 |
| VHH-05 | 7324 | 0.49 | 8095 | 0.53 |
| VHH-06 | 7068 | 0.58 | 8114 | 0.66 |
| P2C47 | 7433 | 0.98 | 8626 | 1.03 |
| 3G8 | 6166 | 1.63 | 8385 | 1.13 |
| NC | 135 | Negative | 204 | Negative |

### 3.2 Effect of human immunoglobulin on the binding of CD16 antibody to cells detected by flow cytometry (fluorescence activated cell sorting, FACS)

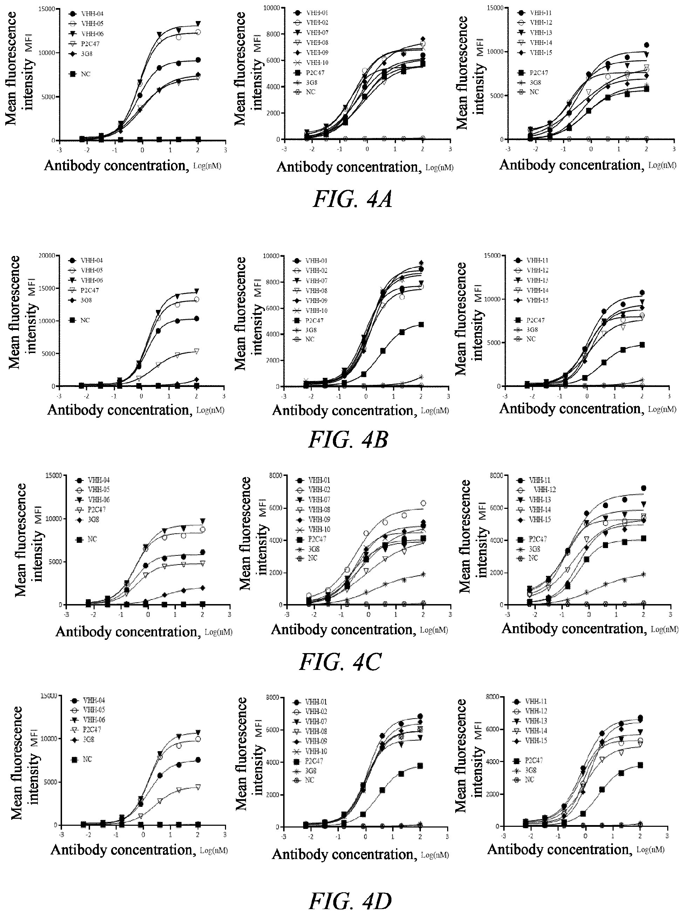

An antibody to be detected was labelled in advance according to the instructions of a biotin labelling kit (EZ-Link^{™} NHS-PEG4 Biotinylation Kit, purchased from Thermofisher Scientific, catalogue number: 21455). The cells were collected, and then directly incubated with the antibody to be detected that had been subjected to gradient dilution, or the cells were first incubated with 10 mg/mL human immunoglobulin (Shanghai Institute of Biological Products Co., Ltd.) and then incubated with the antibody to be detected that had been subjected to gradient dilution. Detection was performed according to the method of Example 3.1, wherein the biotinylated antibody was detected with fluorescein APC-labelled Streptavidin (High Concentration) (purchased from Biolegend, catalogue number: 405243). In the presence of a human immunoglobulin, the binding activity of the control antibody 3G8 to FlpinCHO-human CD16a (158V) was completely lost; the binding activity (EC50) of the VHH antibodies to cells decreased by 2-4 times; the EC50 of the control antibody P2C47 decreased by about 4-5 times; and the results were as shown in FIGS. 4A-4B and Table 11-1. In the presence of a human immunoglobulin, the binding activity of the control antibody 3G8 to FlpinCHO-human CD16a (158F) was completely lost; the binding activity (EC50) of most VHH antibodies to cells decreased by 2-4 times; the EC50 of the control antibody P2C47 decreased by about 7-8 times; and the results were as shown in FIGS. 4C-4D and Table 12-2.

**Table 11-1 Effect of human immunoglobulin on the binding of VHH-Fc antibody to FlpinCHO-CD16a (158V) cell detected by FACS**

| Designation of antibody | FlpinCHO-human CD16a (158V) | | FlpinCHO-human CD16a (158V) +10 mg/mL human immunoglobulin | |
|---|---|---|---|---|
| | Maximum mean fluorescence intensity | EC50 (nM) | Maximum mean fluorescence intensity | EC50 (nM) |
| VHH-01 | 6396 | 0.47 | 9003 | 1.15 |
| VHH-02 | 7297 | 0.33 | 7660 | 1.18 |
| VHH-07 | 5966 | 0.22 | 7903 | 0.85 |
| VHH-08 | 5656 | 0.71 | 8839 | 1.24 |
| VHH-09 | 7638 | 0.64 | 9479 | 1.74 |
| VHH-10 | 6975 | 0.42 | 9195 | 1.39 |
| VHH-11 | 10742 | 0.36 | 10757 | 1.12 |
| VHH-12 | 7951 | 0.16 | 8142 | 0.97 |
| VHH-13 | 9616 | 0.25 | 9660 | 1.09 |
| VHH-14 | 8184 | 0.38 | 7895 | 1.10 |
| VHH-15 | 7256 | 0.49 | 9030 | 1.41 |
| P2C47 | 5741 | 0.58 | 4737 | 3.32 |
| 3G8 | 6175 | 0.70 | 727 | Negative |
| NC | 101 | Negative | 96 | Negative |
| VHH-04 | 9197 | 0.65 | 10363 | 1.51 |
| VHH-05 | 12367 | 0.74 | 13305 | 1.70 |
| VHH-06 | 13285 | 0.79 | 14535 | 1.77 |
| P2C47 | 7149 | 0.77 | 5307 | 2.87 |
| 3G8 | 7517 | 0.99 | 1029 | Negative |
| NC | 134 | Negative | 92 | Negative |

**Table 12-2 Effect of human immunoglobulin on the binding of VHH-Fc antibody to FlpinCHO-CD16a cell detected by FACS**

| Designation of antibody | FlpinCHO-human CD16a (158F) | | FlpinCHO-human CD16a (158F) + 10 mg/mL human immunoglobulin | |
|---|---|---|---|---|
| | Maximum mean fluorescence intensity | EC50 (nM) | Maximum mean fluorescence intensity | EC50 (nM) |
| VHH-01 | 4941 | 0.56 | 6847 | 1.21 |
| VHH-02 | 6308 | 0.33 | 6020 | 1.21 |
| VHH-07 | 4157 | 0.24 | 5525 | 0.87 |
| VHH-08 | 3943 | 0.85 | 6057 | 1.08 |
| VHH-09 | 5145 | 0.39 | 6503 | 1.35 |
| VHH-10 | 4692 | 0.31 | 6041 | 1.20 |
| VHH-11 | 7240 | 0.20 | 6723 | 0.75 |
| VHH-12 | 5410 | 0.12 | 5312 | 0.81 |
| VHH-13 | 6237 | 0.16 | 5829 | 0.67 |
| VHH-14 | 5507 | 0.27 | 5072 | 0.91 |
| VHH-15 | 5239 | 0.40 | 6574 | 1.41 |
| P2C47 | 4136 | 0.41 | 3790 | 2.97 |
| 3G8 | 1924 | 1.73 | 205 | Negative |
| NC | 123 | Negative | 104 | Negative |
| VHH-04 | 6159 | 0.32 | 7585 | 1.51 |
| VHH-05 | 8784 | 0.33 | 9996 | 1.51 |
| VHH-06 | 9742 | 0.39 | 10720 | 1.66 |
| P2C47 | 4847 | 0.39 | 4410 | 2.63 |
| 3G8 | 2009 | 3.43 | 154 | Negative |
| NC | 126 | Negative | 86 | Negative |

### 3.3 Binding of human CD16 antibody to cells expressing different mutants of CD16b detected by flow cytometry (fluorescence activated cell sorting, FACS)

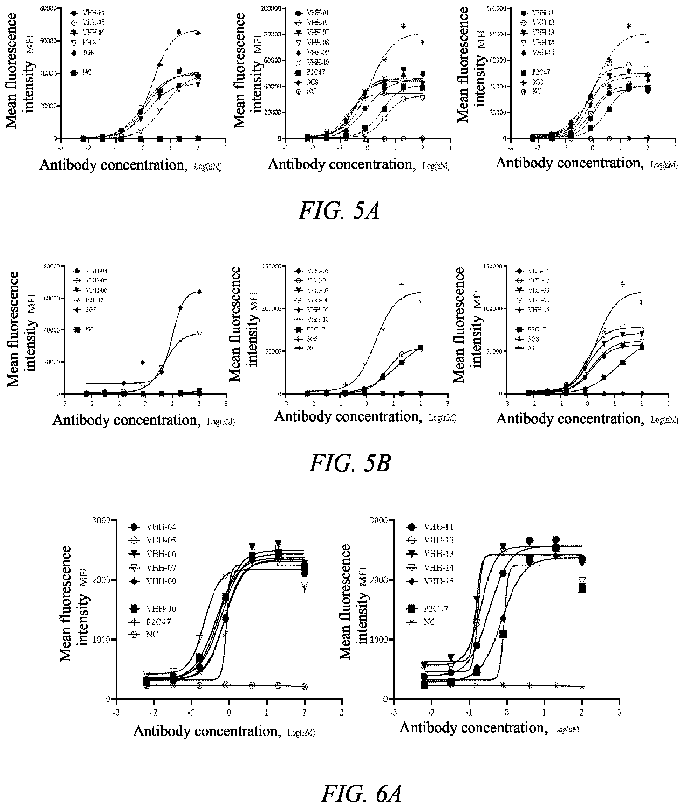

The binding of an antibody to be detected to FlpinCHO-human CD16b cells was detected according to the method of Example 3.1. As shown in FIGS. 5A-5B and Table 12, the 3G8 antibody was a positive antibody binding to CD16b, and the binding activity of all the antibodies to be detected to FlpinCHO-human CD16b (NA1) and FlpinCHO-human CD16b (NA2) was weaker than that of the 3G8 antibody.

**Table 13 Binding activity of VHH-Fc antibody with FlpinCHO-CD16b cell detected by FACS**

| Designation of antibody | FlpinCHO-human CD16b (NA1) | | FlpinCHO-human CD16b (NA2) | |
|---|---|---|---|---|
| | Maximum mean fluorescence intensity | EC50 (nM) | Maximum mean fluorescence intensity | EC50 (nM) |
| VHH-01 | 49685 | 0.85 | 95 | Negative |
| VHH-02 | 31948 | 4.27 | 52411 | 5.45 |
| VHH-07 | 52884 | 0.35 | 113 | Negative |
| VHH-08 | 38590 | 0.18 | 149 | Negative |
| VHH-09 | 48217 | 0.30 | 143 | Negative |
| VHH-10 | 51401 | 0.34 | 140 | Negative |
| VHH-11 | 37891 | 1.03 | 56883 | 1.43 |
| VHH-12 | 58080 | 0.62 | 79053 | 0.96 |
| VHH-13 | 51447 | 0.77 | 70183 | 1.11 |
| VHH-14 | 41885 | 0.93 | 61412 | 1.44 |
| VHH-15 | 52065 | 0.44 | 178 | Negative |
| P2C47 | 40376 | 3.18 | 54582 | 14.2 |
| 3G8 | 86380 | 1.22 | 129030 | 2.13 |
| NC | 577 | Negative | 577 | Negative |
| VHH-04 | 41352 | 1.33 | 351 | Negative |
| VHH-05 | 42465 | 0.96 | 2132 | Negative |
| VHH-06 | 33268 | 1.41 | 1934 | Negative |
| P2C47 | 37921 | 5.50 | 37456 | 5.12 |
| 3G8 | 64741 | 2.13 | 63891 | 9.80 |
| NC | 322 | Negative | 91 | Negative |

### Example 4 Detection of binding activity of CD16 antibody to primary human NK cell

### 4.1 Binding of human CD16 antibody to resting NK cells detected by flow cytometry (fluorescence activated cell sorting, FACS)

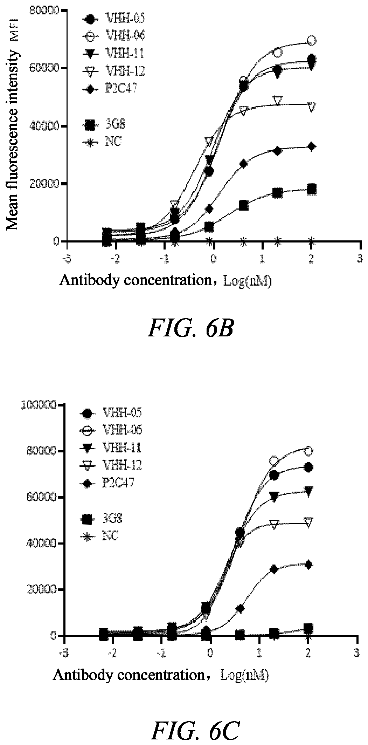

The binding of an antibody to be detected to freshly prepared human NK cells (Milestone Biotechnologies, catalogue number: PB56-N-Custom) was detected according to the method of Example 3.1. As shown in FIG. 6A and Table 13, all antibodies detected had good binding activity to NK cells, and the EC50 was less than 1 nM.

**Table 14 Binding activity of VHH-hFc antibody with NK cell detected by FACS**

| Designation of antibody | NK cell | |
|---|---|---|
| | Maximum mean fluorescence intensity | EC50 (nM) |
| VHH-04 | 2430 | 0.74 |
| VHH-05 | 2556 | 0.62 |
| VHH-06 | 2605 | 0.54 |
| VHH-07 | 2332 | 0.22 |
| VHH-09 | 2425 | 0.76 |
| VHH-10 | 2450 | 0.45 |
| VHH-11 | 2664 | 0.33 |
| VHH-12 | 2680 | 0.21 |
| VHH-13 | 2627 | 0.15 |
| VHH-14 | 2618 | 0.17 |
| VHH-15 | 2397 | 0.74 |
| P2C47 | 2535 | 0.84 |
| NC | 238 | Negative |

### 4.2 Effect of human immunoglobulin presence on the binding of human CD16 antibody to resting NK cells detected by flow cytometry (fluorescence activated cell sorting, FACS)

Detection was performed according to the method of Example 3.2, and the results were as shown in FIG. 6B and Table 14. In the presence of a human immunoglobulin, the binding activity of the control antibody 3G8 to NK cells was completely lost; the binding activity (EC50) of the antibodies VHH-05, VHH-06 and VHH-11 to cells decreased by 2-3 times; and the EC50 of the control antibody P2C47 decreased by about 4 times. Moreover, in the presence of a human immunoglobulin, the binding activity of VHH-05, VHH-06, VHH-11 and VHH-12 to NK cells was stronger than that of the P2C47 antibody.

**Table 15 Binding activity of VHH-hFc antibody with NK cell detected by FACS in the presence of human immunoglobulin**

| Designation of antibody | NK cell | | NK cell +10 mg/mL human immunoglobulin | |
|---|---|---|---|---|
| | Maximum mean fluorescence intensity | EC50 (nM) | Maximum mean fluorescence intensity | EC50 (nM) |
| VHH-05 | 63193 | 1.15 | 72850 | 2.94 |
| VHH-06 | 69615 | 1.42 | 80047 | 3.9 |
| VHH-11 | 60694 | 0.95 | 62256 | 2.31 |
| VHH-12 | 46459 | 0.40 | 48943 | 1.82 |
| P2C47 | 32972 | 1.33 | 30926 | 5.4 |
| 3G8 | 18076 | 1.99 | 3473 | Negative |
| NC | 87 | Negative | 87 | Negative |

### Example 5 Detection of cross-species activity of CD16 antibody

### 5.1 Binding of CD16 antibody to cell expressing cynomolgus monkey CD16 detected by FACS

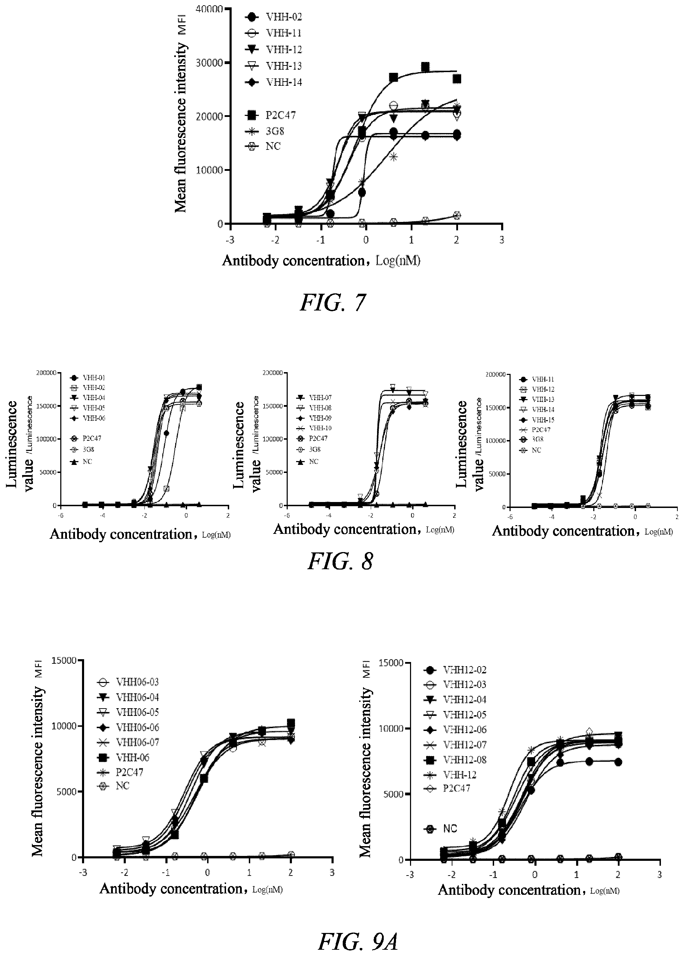

Flipin CHO-cyno CD16 cells were subjected to FACS detection and data analysis according to the method of Example 3.1. The analysis results were as shown in Table 15 and FIG. 7. The antibodies VHH-11, VHH-12, VHH-13 and VHH-I4 all had binding activity to Flipin CHO cells overexpressing cyno CD16, and the EC50 showed that the highest binding activity was 0.18 nM.

**Table 16 Binding activity of VHH-hFc antibody with FlpinCHO-cyno CD16 cell detected by FACS**

| Designation of antibody | FlpinCHO-cyno CD16 cell | |
|---|---|---|
| | Maximum mean fluorescence intensity | EC50 (nM) |
| VHH02 | 17078 | 0.88 |
| VHH11 | 22013 | 0.41 |
| VHH12 | 22153 | 0.24 |
| VHH13 | 21391 | 0.24 |
| VHH14 | 16377 | 0.18 |
| P2C47 | 29209 | 0.58 |
| 3G8 | 22346 | 2.92 |
| NC | 1569 | Negative |

### Example 6 Functional identification of human CD16 antibody

### 6.1 Function of human CD16 antibody detected by luciferase report assay

A Jurkat-NFAT luciferase reporter cell line (purchased from BPS Bioscience, catalogue number: 60541) stably expressing human CD16a (158V) was used to detect the signalling pathway in the luciferase reporter cell line activated by a CD16 antibody. The signal detection of luciferase was carried out according to the operating instructions of Bright-Glo^{™} Luciferase Assay System kit (Promega, catalogue number: E2620). The experimental method was as follows: 2 µg/mL Fc-tagged anti-human antibody (Jackson Immune, catalogue number: 109-006-098) was coated overnight at 4°C to capture an antibody to be tested with a human Fc tag. The next day, the antibody to be detected was diluted with RPMI 1640 medium containing 5% FBS, with an initial antibody concentration of 4 nM and gradient dilution of 1 : 6. Subsequently, the antibody was added to a 96-well flat-bottomed cell culture plate at 50 µl/well, and pre-incubated at 37°C for 15 minutes. After incubation, the collected luciferase reporter cell line in the logarithmic growth phase was adjusted to the cell concentration of 8 × 10⁵/mL with a medium (RPMI 1640, purchased from Gibco, catalogue number: 12633012) containing 2% FBS, and the cells were added to the culture plate at 50 µl/well. The cells were incubated in a 37°C incubator for 6 hours and then centrifuged at 400 g for 5 minutes. 20 µL of the supernatant was added to a black opaque 96-well detection plate (Greiner, catalogue number: 655090), and 50 µL of a detection reagent was added. The response value was read by PerkiElmer Ensight-HH3400 microplate reader, and then software (GraphPad Prism8) was used for analysis, data fitting and EC50 value calculation. The results were as shown in Table 16 and FIG. 8. In the report assay, all antibodies could activate the downstream signalling pathway of Jurkat cells, and the activation activity was comparable to or stronger than that by the P2C47 antibody.

**Table 17 Activation activity by CD16 antibody detected by luciferase report assay**

| Designation of antibody | Activation activity for Jurkat cell | |
|---|---|---|
| | Maximum fluorescence value | EC50 (nM) |
| VHH01 | 177855 | 0.09 |
| VHH02 | 178360 | 0.30 |
| VHH07 | 173550 | 0.02 |
| VHH08 | 178405 | 0.02 |
| VHH09 | 159005 | 0.03 |
| VHH10 | 156650 | 0.02 |
| VHH11 | 165875 | 0.03 |
| VHH12 | 165370 | 0.02 |
| VHH13 | 164735 | 0.02 |
| VHH14 | 168680 | 0.02 |
| VHH15 | 166735 | 0.02 |
| P2C47 | 157890 | 0.04 |
| 3G8 | 153110 | 0.03 |
| NC | 2070 | Negative |
| VHH-04 | 160915 | 0.03 |
| VHH-05 | 170720 | 0.03 |
| VHH-06 | 169215 | 0.04 |

### Example 7 Affinity assay of CD16 antibody

### 7.1 Affinity assay of CD16 antibody to human CD16a-ECD-his protein

Anti-human CD16 VHH-hFc antibody was captured using a Protein A chip (GE Healthcare; 29-127-558). A sample buffer and a running buffer were HBS-EP+ (10 mM HEPES, 150 mM NaCl, 3 mM EDTA and 0.05% surfactant P20) (GE Healthcare; BR-1006-69). The flow-through cell was set to 25°C. The sample block was set to 16°C. Both were pretreated with the running buffer. In each cycle, an antibody to be detected was first captured with a Protein A chip, and then a single concentration of CD16 antigen protein was injected. The binding and dissociation processes of the antibody and the antigen protein were recorded, and finally Glycine pH 1.5 (GE Healthcare; BR-1003-54) was used to complete chip regeneration. Binding was measured by injecting different concentrations of recombinant human CD16-ECD his proteins in a solution for 240 s with a flow rate of 30 µL/min, wherein a total of 5 concentrations were prepared by using 200 nM as a starting concentration (see the detailed results for the actual concentration in the test) and performing a 1:1 dilution. The dissociation phase was monitored for up to 600 s and was triggered by switching from a sample solution to a running buffer. The surface was regenerated by washing with a 10 mM glycine solution (pH 1.5) for 30 s at a flow rate of 30 µL/min. Bulk refractive index difference was corrected by subtracting the response obtained from the goat anti-human Fc surface. Blank injection was also subtracted (= double reference). For calculation of apparent KD and other kinetic parameters, Langmuir 1 : 1 model was used. The binding rate (Ka), dissociation rate (Kd) and binding affinity (KD) of the antibodies to be detected to human CD16-ECD his proteins were as shown in Table 17.

**Table 18 Binding of CD16 antibody to human CD16a protein detected by Biacore**

| Designation of antibody | Human CD16 a (158F) | | | Human CD16 a (158V) | | |
|---|---|---|---|---|---|---|
| | ka (1/Ms) | kd (1/s) | KD (M) | ka (1/Ms) | kd (1/s) | KD (M) |
| VHH01 | 7.66E+04 | 8.31E-03 | 1.08E-07 | 1.38E+05 | 3.73E-03 | 2.70E-08 |
| VHH02 | 2.05E+05 | 2.70E-02 | 1.32E-07 | 1.86E+05 | 1.79E-02 | 9.65E-08 |
| VHH04 | 5.47E+04 | 1.28E-03 | 2.34E-08 | 8.26E+04 | 7.38E-04 | 8.93E-09 |
| VHH05 | 6.12E+04 | 4.99E-04 | 8.16E-09 | 8.61E+04 | 3.21E-04 | 3.73E-09 |
| VHH06 | 6.40E+04 | 3.74E-04 | 5.84E-09 | 1.09E+05 | 2.13E-04 | 1.96E-09 |
| VHH07 | 6.72E+04 | 4.51E-03 | 6.72E-08 | 1.10E+05 | 2.49E-03 | 2.27E-08 |
| VHH08 | 6.95E+04 | 5.59E-03 | 8.05E-08 | 1.20E+05 | 2.66E-03 | 2.21E-08 |
| VHH09 | 6.16E+04 | 3.09E-03 | 5.02E-08 | 1.10E+05 | 1.78E-03 | 1.61E-08 |
| VHH10 | 6.18E+04 | 2.47E-03 | 3.99E-08 | 1.07E+05 | 1.46E-03 | 1.36E-08 |
| VHH11 | 5.48E+05 | 2.35E-02 | 4.29E-08 | 1.53E+06 | 4.91E-02 | 3.20E-08 |
| VHH12 | 8.78E+05 | 1.17E-02 | 1.33E-08 | 1.86E+06 | 2.01E-02 | 1.08E-08 |
| VHH13 | 8.66E+05 | 1.92E-02 | 2.22E-08 | 1.83E+06 | 3.62E-02 | 1.98E-08 |
| VHH14 | 1.18E+06 | 2.96E-02 | 2.50E-08 | 3.12E+06 | 6.02E-02 | 1.93E-08 |
| VHH15 | 6.38E+04 | 2.73E-03 | 4.28E-08 | 1.14E+05 | 1.60E-03 | 1.41E-08 |

### 7.2 Affinity assay of CD16 antibody to human CD16b-ECD-his protein

The binding rate (ka), dissociation rate (kd) and binding affinity (KD) of the antibodies to be tested to human CD16b-ECD his proteins were detected according to the method of Example 7.1, as shown in Table 18 and Table 19.

**Table 19 Binding of CD16 antibody to human CD16b protein detected by Biacore**

| Designation of antibody | Human CD16 b (NA1) | | |
|---|---|---|---|
| | ka (1/Ms) | kd (1/s) | KD (M) |
| VHH01 | 5.07E+05 | 4.85E-02 | 9.57E-08 |
| VHH04 | 1.22E+05 | 2.65E-03 | 2.16E-08 |
| VHH05 | 1.03E+05 | 9.58E-04 | 9.28E-09 |
| VHH06 | 1.30E+05 | 1.13E-03 | 8.63E-09 |
| VHH11 | 4.31E+04 | 2.59E-01 | 6.01E-06 |
| VHH12 | 1.03E+05 | 1.54E-01 | 1.50E-06 |

**Table 20 Binding of CD16 antibody to human CD16b protein detected by Biacore**

| Designation of antibody | Human CD16 b (NA2) | | | Human CD16 b (SH) | | |
|---|---|---|---|---|---|---|
| | ka (1/Ms) | kd (1/s) | KD (M) | ka (1/Ms) | kd (1/s) | KD (M) |
| VHH11 | 4.07E+04 | 2.00E-01 | 4.92E-06 | 3.91E+04 | 1.93E-01 | 4.93E-06 |
| VHH12 | 9.51E+04 | 9.98E-02 | 1.05E-06 | 9.15E+04 | 1.06E-01 | 1.16E-06 |

### 7.3 Affinity assay of CD16 antibody to cyno CD16-ECD-his protein

The binding rate (ka), dissociation rate (kd) and binding affinity (KD) of the antibodies to be tested to cyno CD16-ECD his proteins were detected according to the method of Example 7.1, as shown in Table 20.

**Table 21 Binding of CD16 antibody to cyno CD16 protein detected by Biacore**

| Designation of antibody | Cyno CD16 | | |
|---|---|---|---|
| | ka (1/Ms) | kd (1/s) | KD (M) |
| VHH-11 | 6.33E+05 | 1.50E-02 | 2.36E-08 |
| VHH-12 | 1.18E+06 | 1.01E-02 | 8.51E-09 |

### Example 8 Humanized design and preparation of anti-CD16 single domain antibody

By comparing the IMGT (http://imgt.cines.fr) human antibody heavy and light chain variable region germline gene database, IGHV3-30*02 and IGHV3-7*01 were selected as humanized heavy chain templates for llama antibodies VHH-06 and VHH-12, respectively. First, the CDRs of llama antibodies were grafted into the FRs of the human templates thereof, respectively, to form a variable region sequence in the order of FR1-CDR1-FR2-CDR2-FR3-CDR3-FR4. According to the three-dimensional structure simulation of the antibody, the key amino acids in the FR region sequence of the humanized antibody were backmutated to the corresponding amino acids of the llama antibody to ensure the original affinity, and a humanized anti-CD 16 single domain antibody was obtained. The numbering of amino acid residues and the CDR regions of the antibody in this example were determined and annotated by the IMGT numbering system (see http://www.abysis.org/abysis/sequence_input/key_annotation/key_annotation.cgi for details). The humanized antibody VHHs designed by the above-mentioned method were as shown in Table 21 and Table 22, respectively.

**Table 21 Design of humanized VH of VHH06**

| **VH** | **FR Template** | **FR mutations** |
|---|---|---|
| VH3 | IGHV3-30*02 | M39V,H40R,V42Y,G49Q,L50R,Y66N,L87V,V101D,Y103I |
| VH4 | IGHV3-30*02 | M39V,H40R,V42Y,G49Q,L50R,Y66N,A68P,L87V,V101D,Y103I |
| VH5 | IGHV3-30*02 | M39V,H40R,V42Y,G49Q,L50R,Y66N,S83A,L87V,V101D,Y103I |
| VH6 | IGHV3-30*02 | M39V,H40R,V42Y,G49Q,L50R,Y66N,I78V,L87V,V101D,Y103I |
| VH7 | IGHV3-30*02 | M39V,H40R,V42Y,G49Q,L50R,Y66N,L87V,V101D,Y103I,M123Q |

| | | |
|---|---|---|
| Notes: M39V means that M at position 39 of Graft is mutated into V, and so on. The numbering of backmutated amino acids is according to the IMGT numbering. | | |

**Table 22 Design of humanized VH of VHH12**

| **VH** | **FR Template** | **FR mutations** |
|---|---|---|
| VH2 | IGHV3-30*01 | S40G,V42Y,G49Q,L50R,W52L,N55A |
| VH3 | IGHV3-30*01 | S40G,V42Y,G49Q,L50R,W52L,N55A,Y66N |
| VH4 | IGHV3-30*01 | S40G,V42Y,G49Q,L50R,W52L,N55A,Y66N,V68A,S70F |
| VH5 | IGHV3-30*01 | S40G,V42Y,G49Q,L50R,W52L,N55A,Y66N,D81V |
| VH6 | IGHV3-30*01 | S40G,V42Y,G49Q,L50R,W52L,N55A,Y66N,L87V |
| VH7 | IGHV3-30*01 | S40G,V42Y,G49Q,L50R,W52L,N55A,Y66N,M123Q |
| VH8 | IGHV3-30*01 | S40G,V42Y,G49Q,L50R,W52L,N55A,Y66N,V68A,S70F,D81V |

| | | |
|---|---|---|
| Notes: S40G means that S at position 40 of Graft is mutated into G, and so on. The numbering of backmutated amino acids is according to the IMGT numbering. | | |

The humanized VHH sequences were as shown in Table 23 and Table 24. The humanized antibody sequences were used to prepare VHH-Fc antibodies according to the method of Example 1.2, and the humanized VHH-Fc sequences were as shown in Table 25 and Table 26.

**Table 23 Sequence of VHH06 humanized antibody**

| **Designati on of sequence** | **Sequen ce number** | **Amino acid sequence** |
|---|---|---|
| VHH06-03 | SEQ ID NO: 90 | EVQLVESGGGVVQPGGSLRLSCAASGSTWTTYAVRWYRQAPGKQ REWVAFISVDGSTNYADSVKGRFTISRDNSKNTVYLQMNSLRAED TADYICRVGGQYWGQGTMVTVSS |
| VHH06-04 | SEQ ID NO: 91 | EVQLVESGGGVVQPGGSLRLSCAASGSTWTTYAVRWYRQAPGKQ REWVAFISVDGSTNYPDSVKGRFTISRDNSKNTVYLQMNSLRAEDT ADYICRVGGQYWGQGTMVTVSS |
| VHH06-05 | SEQ ID NO: 92 | EVQLVESGGGVVQPGGSLRLSCAASGSTWTTYAVRWYRQAPGKQ REWVAFISVDGSTNYADSVKGRFTISRDNAKNTVYLQMNSLRAED TADYICRVGGQYWGQGTMVTVSS |
| VHH06-06 | SEQ ID NO: 93 | EVQLVESGGGVVQPGGSLRLSCAASGSTWTTYAVRWYRQAPGKQ REWVAFISVDGSTNYADSVKGRFTVSRDNSKNTVYLQMNSLRAED TADYICRVGGQYWGQGTMVTVSS |
| VHH06-07 | SEQ ID NO: 94 | EVQLVESGGGVVQPGGSLRLSCAASGSTWTTYAVRWYRQAPGKQ REWVAFISVDGSTNYADSVKGRFTISRDNSKNTVYLQMNSLRAED TADYICRVGGQYWGQGTQVTVSS |

**Table 24 Sequence of VHH12 humanized antibody**

| **Designati on of sequence** | **Sequence number** | **Amino acid sequence** |
|---|---|---|
| VHH12-02 | SEQ ID NO: 95 | EVQLVESGGGLVQPGGSLRLSCAASGITFSSYAMGWYRQAPGKQ RELVAAINRFGLTYYVDSVKGRFTISRDNAKNSLYLQMNSLRAED TAVYYCNTGGVYSHPDYYTGTYWGQGTMVTVSS |
| VHH12-03 | SEQ ID NO: 96 | EVQLVESGGGLVQPGGSLRLSCAASGITFSSYAMGWYRQAPGKQ RELVAAINRFGLTNYVDSVKGRFTISRDNAKNSLYLQMNSLRAED TAVYYCNTGGVYSHPDYYTGTYWGQGTMVTVSS |
| VHH12-04 | SEQ ID NO: 97 | EVQLVESGGGLVQPGGSLRLSCAASGITFSSYAMGWYRQAPGKQ RELVAAINRFGLTNYADFVKGRFTISRDNAKNSLYLQMNSLRAED TAVYYCNTGGVYSHPDYYTGTYWGQGTMVTVSS |
| VHH12-05 | SEQ ID NO: 98 | EVQLVESGGGLVQPGGSLRLSCAASGITFSSYAMGWYRQAPGKQ RELVAAINRFGLTNYVDSVKGRFTISRVNAKNSLYLQMNSLRAED |
| | | TAVYYCNTGGVYSHPDYYTGTYWGQGTMVTVSS |
| VHH12-06 | SEQ ID NO: 99 | EVQLVESGGGLVQPGGSLRLSCAASGITFSSYAMGWYRQAPGKQ RELVAAINRFGLTNYVDSVKGRFTISRDNAKNSVYLQMNSLRAED TAVYYCNTGGVYSHPDYYTGTYWGQGTMVTVSS |
| VHH12-07 | SEQ ID NO: 100 | EVQLVESGGGLVQPGGSLRLSCAASGITFSSYAMGWYRQAPGKQ RELVAAINRFGLTNYVDSVKGRFTISRDNAKNSLYLQMNSLRAED TAVYYCNTGGVYSHPDYYTGTYWGQGTQVTVSS |
| VHH12-08 | SEQ ID NO: 101 | EVQLVESGGGLVQPGGSLRLSCAASGITFSSYAMGWYRQAPGKQ RELVAAINRFGLTNYADFVKGRFTISRVNAKNSLYLQMNSLRAED TAVYYCNTGGVYSHPDYYTGTYWGQGTMVTVSS |

**Table 25 Sequence of VHH06-Fc humanized antibody**

| **Designat ion of sequence** | **Sequ ence num ber** | **Amino acid sequence** |
|---|---|---|
| VHH06-03 | SEQ ID NO: 102 | EVQLVESGGGVVQPGGSLRLSCAASGSTWTTYAVRWYRQAPGKQR EWVAFISVDGSTNYADSVKGRFTISRDNSKNTVYLQMNSLRAEDTA DYICRVGGQYWGQGTMVTVSSEPKSSDKTHTCPPCPAPEEFGGPS VFLFPPKPKDTLMISRTPEVTCVVVAVSHEDPEVKFNWYVDGVE VHNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH06-04 | SEQ ID NO: 103 | EVQLVESGGGVVQPGGSLRLSCAASGSTWTTYAVRWYRQAPGKQR EWVAFISVDGSTNYPDSVKGRFTISRDNSKNTVYLQMNSLRAEDTA DYICRVGGQYWGQGTMVTVSSEPKSSDKTHTCPPCPAPEEFGGPS VFLFPPKPKDTLMISRTPEVTCVVVAVSHEDPEVKFNWYVDGVE VHNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH06-05 | SEQ ID NO: 104 | EVQLVESGGGVVQPGGSLRLSCAASGSTWTTYAVRWYRQAPGKQR EWVAFISVDGSTNYADSVKGRFTISRDNAKNTVYLQMNSLRAEDTA DYICRVGGQYWGQGTMVTVSSEPKSSDKTHTCPPCPAPEEFGGPS VFLFPPKPKDTLMISRTPEVTCVVVAVSHEDPEVKFNWYVDGVE VHNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR |
| | | WQQGNVFSCSVMHEALHNHYTQKSLSLSPGK |
| VHH06-06 | SEQ ID NO: 105 | EVQLVESGGGVVQPGGSLRLSCAASGSTWTTYAVRWYRQAPGKQR EWVAFISVDGSTNYADSVKGRFTVSRDNSKNTVYLQMNSLRAEDTA DYICRVGGQYWGQGTMVTVSSEPKSSDKTHTCPPCPAPEEFGGPS VFLFPPKPKDTLMISRTPEVTCVVVAVSHEDPEVKFNWYVDGVE VHNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |
| VHH06-07 | SEQ ID NO: 106 | EVQLVESGGGVVQPGGSLRLSCAASGSTWTTYAVRWYRQAPGKQR EWVAFISVDGSTNYADSVKGRFTISRDNSKNTVYLQMNSLRAEDTA DYICRVGGQYWGQGTQVTVSSEPKSSDKTHTCPPCPAPEEFGGPS VFLFPPKPKDTLMISRTPEVTCVVVAVSHEDPEVKFNWYVDGVE VHNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKSR WQQGNVFSCSVMHEALHNHYTQKSLSLSPGK |

| | | |
|---|---|---|
| Notes: the bold font is the human hIgG1 Fc sequence. | | |

**Table 26 Sequence of VHH12-Fc humanized antibody**

| **Designat ion of sequence** | **Sequence number** | **Amino acid sequence** |
|---|---|---|
| VHH12-02 | SEQ ID NO: 107 | EVQLVESGGGLVQPGGSLRLSCAASGITFSSYAMGWYRQAPGKQ RELVAAINRFGLTYYVDSVKGRFTISRDNAKNSLYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |
| VHH12-03 | SEQ ID NO: 108 | EVQLVESGGGLVQPGGSLRLSCAASGITFSSYAMGWYRQAPGKQ RELVAAINRFGLTNYVDSVKGRFTISRDNAKNSLYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP |
| | | PVLDSDGSFFLYSKLTVDKSRWQQGNVFSCSVMHEALHNHYT QKSLSLSPGK |
| VHH12-04 | SEQ ID NO: 109 | EVQLVESGGGLVQPGGSLRLSCAASGITFSSYAMGWYRQAPGKQ RELVAAINRFGLTNYADFVKGRFTISRDNAKNSLYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |
| VHH12-05 | SEQ ID NO: 110 | EVQLVESGGGLVQPGGSLRLSCAASGITFSSYAMGWYRQAPGKQ RELVAAINRFGLTNYVDSVKGRFTISRVNAKNSLYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |
| VHH12-06 | SEQ ID NO: 111 | EVQLVESGGGLVQPGGSLRLSCAASGITFSSYAMGWYRQAPGKQ RELVAAINRFGLTNYVDSVKGRFTISRDNAKNSVYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |
| VHH12-07 | SEQ ID NO: 112 | EVQLVESGGGLVQPGGSLRLSCAASGITFSSYAMGWYRQAPGKQ RELVAAINRFGLTNYVDSVKGRFTISRDNAKNSLYLQMNSLRAED TAVYYCNTGGVYSHPDYYTGTYWGQGTQVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |
| VHH12-08 | SEQ ID NO: 113 | EVQLVESGGGLVQPGGSLRLSCAASGITFSSYAMGWYRQAPGKQ RELVAAINRFGLTNYADFVKGRFTISRVNAKNSLYLQMNSLRAED TAVYYCNTGGVYSHPDYYTGTYWGQGTMVTVSSEPKSSDKTHT CPPCPAPEEFGGPSVFLFPPKPKDTLMISRTPEVTCVVVAVSHE DPEVKFNWYVDGVEVHNAKTKPREEQYNSTYRVVSVLTVLH QDWLNGKEYKCKVSNKALPAPIEKTISKAKGQPREPQVYTLP PSREEMTKNQVSLTCLVKGFYPSDIAVEWESNGQPENNYKTTP PVLDSDGSFFLYSKLTVDKSRWQQGNVFSCSVMHEALHNHYT QKSLSLSPGK |

| | | |
|---|---|---|
| Notes: the bold font is the human hIgG1 Fc sequence. | | |

### Example 9 Detection of binding activity of CD16 antibody

### 9.1 Binding of human CD16 antibody to cells expressing different mutants of CD16a detected by flow cytometry (fluorescence activated cell sorting, FACS)

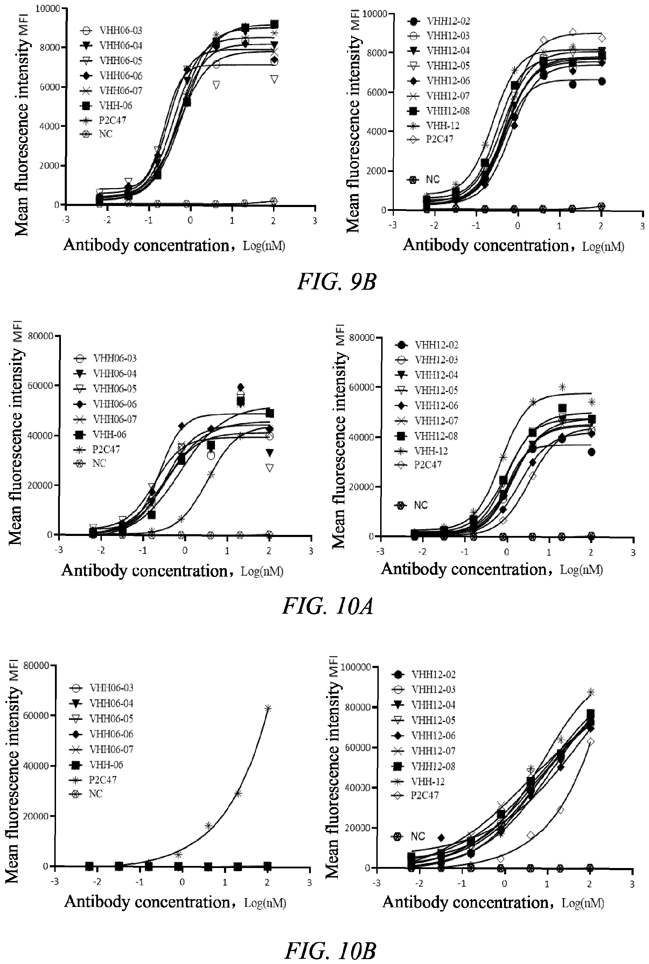

The binding of a humanized antibody to FlpinCHO-human CD16a cells was detected according to the method of Example 3.1. As shown in FIGS. 9A-9B and Table 27, the humanized antibodies had good binding activity to both FlpinCHO-CD16a (158F) and FlpinCHO-CD16a (158V) cells, and had comparable EC50 in terms of binding to the two cells, with EC50 less than 1 nM.

**Table 27 Binding activity of VHH-Fc antibody with FlpinCHO-CD16a cell detected by FACS**

| Designation of antibody | FlpinCHO-human CD16a (158F) | | FlpinCHO-human CD16a (158V) | |
|---|---|---|---|---|
| | Maximum mean fluorescence intensity | EC50 (nM) | Maximum mean fluorescence intensity | EC50 (nM) |
| VHH06-03 | 9143 | 0.47 | 8394 | 0.49 |
| VHH06-04 | 9572 | 0.39 | 8893 | 0.40 |
| VHH06-05 | 8890 | 0.27 | 8839 | 0.23 |
| VHH06-06 | 8934 | 0.30 | 8242 | 0.28 |
| VHH06-07 | 9157 | 0.50 | 8458 | 0.50 |
| VHH-06 | 10227 | 0.61 | 9247 | 0.66 |
| VHH12-02 | 7438 | 0.46 | 6603 | 0.45 |
| VHH 12-03 | 8814 | 0.48 | 7751 | 0.48 |
| VHH 12-04 | 9407 | 0.47 | 8091 | 0.47 |
| VHH 12-05 | 9345 | 0.35 | 8129 | 0.41 |
| VHH 12-06 | 8767 | 0.60 | 7546 | 0.64 |
| VHH 12-07 | 9177 | 0.46 | 7947 | 0.48 |
| VHH12-08 | 9011 | 0.31 | 7923 | 0.32 |
| VHH-12 | 8730 | 0.23 | 8046 | 0.24 |
| P2C47 | 9400 | 0.56 | 8801 | 0.54 |
| NC | 216 | Negative | 248 | Negative |

### 9.2 Binding of human CD16 antibody to cells expressing different mutants of CD16b detected by flow cytometry (fluorescence activated cell sorting, FACS)

The binding of an antibody to be detected to FlpinCHO-human CD16b cells was detected according to the method of Example 3.1. It had been detected in Example 3 that the binding activity of P2C47 to FlpinCHO-human CD16b (NA1) and FlpinCHO-human CD16b (NA1) was significantly weaker than that of the CD16a and CD16b positive antibody 3G8. As shown in FIGS. 10A-10B and Table 28, the binding of humanized VHH antibodies to FlpinCHO-human CD16b (NA1) and FlpinCHO-human CD16b (NA2) was comparable to that of the control antibody P2C47, so it could be speculated that the binding activity of humanized antibodies was also weaker than that of the 3G8 antibody.

**Table 28 Binding activity of VHH-Fc antibody with FlpinCHO-CD16b cell detected by FACS**

| Designation of antibody | FlpinCHO-human CD16b (NA1) | | FlpinCHO-human CD16b (NA2) | |
|---|---|---|---|---|
| | Maximum mean fluorescence intensity | EC50 (nM) | Maximum mean fluorescence intensity | EC50 (nM) |
| VHH06-03 | 56938 | 0.35 | 174 | Negative |
| VHH06-04 | 53033 | 0.32 | 437 | Negative |
| VHH06-05 | 55236 | 0.18 | 296 | Negative |
| VHH06-06 | 59750 | 0.25 | 204 | Negative |
| VHH06-07 | 53477 | 0.33 | 167 | Negative |
| VHH-06 | 54107 | 0.74 | 468 | Negative |
| VHH12-02 | 39675 | 0.94 | 73319 | NA |
| VHH12-03 | 47320 | 1.37 | 72714 | NA |
| VHH12-04 | 47184 | 1.37 | 75390 | NA |
| VHH12-05 | 48199 | 0.95 | 74036 | NA |
| VHH12-06 | 40566 | 1.94 | 69819 | NA |
| VHH12-07 | 45525 | 1.25 | 73176 | NA |
| VHH12-08 | 52066 | 0.98 | 77253 | NA |
| VHH-12 | 60475 | 0.70 | 87921 | NA |
| P2C47 | 43566 | 3.36 | 63364 | NA |
| NC | 189 | Negative | 447 | Negative |

| | | | | |
|---|---|---|---|---|
| Notes: NA indicates weak antibody binding activity and poor curve fitting. | | | | |

### Example 10 Detection of binding activity of CD16 antibody to primary human NK cell and Granulocytes

### 10.1 Effect of human immunoglobulin presence on the binding of human CD16 antibody to resting NK cells detected by flow cytometry (fluorescence activated cell sorting, FACS)

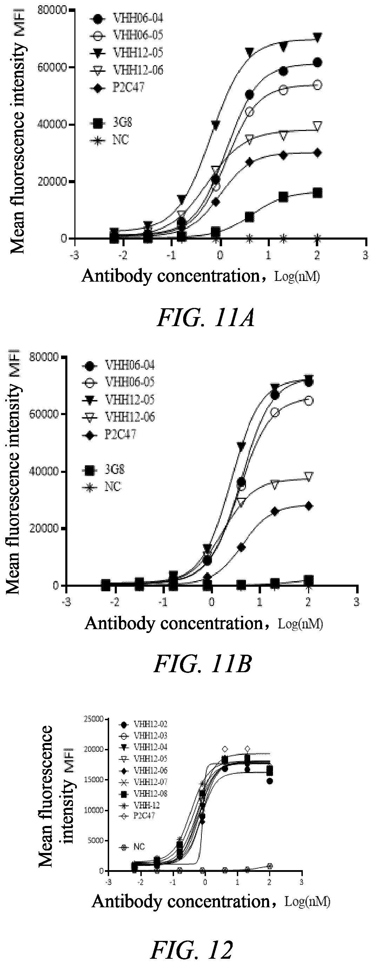

Detection was performed according to the method of Example 3.2, and the results were as shown in FIGS. 11A-11B and Table 29. In the presence of a human immunoglobulin, the binding activity of the control antibody 3G8 to NK cells was completely lost; the the binding activity (EC50) of the antibodies VHH05, VHH06 and VHH11 to cells decreased by 3-4 times; and the EC50 of the control antibody P2C47 decreased by about 4-5 times. Moreover, in the presence of a human immunoglobulin, the binding activity of VHH06-03, VHH06-04, VHH12-05 and VHH12-06 to NK cells was stronger than that of the P2C47 antibody.

**Table 29 Binding activity of VHH-hFc antibody with NK cell detected by FACS in the presence of human immunoglobulin**

| Designation of antibody | NK cell | | NK cell +10 mg/mL human immunoglobulin | |
|---|---|---|---|---|
| | Maximum mean fluorescence intensity | EC50 (nM) | Maximum mean fluorescence intensity | EC50 (nM) |
| VHH06-04 | 61811 | 1.34 | 71433 | 3.99 |
| VHH06-05 | 53923 | 1.33 | 64861 | 3.66 |
| VHH12-05 | 70398 | 0.65 | 72108 | 2.48 |
| VHH12-06 | 39262 | 0.53 | 38118 | 1.69 |
| P2C47 | 30136 | 0.99 | 27971 | 4.30 |
| 3G8 | 16008 | 4.45 | 1963 | Negative |
| NC | 103 | Negative | 105 | Negative |

### Example 11 Detection of cross-species activity of CD16 antibody

### 11.1 Binding of CD16 antibody to cell expressing cynomolgus monkey CD16 detected by FACS

Flipin CHO-cyno CD16 cells were subjected to FACS detection and data analysis according to the method of Example 3.1. The analysis results were as shown in Table 30 and FIG. 12. All humanized antibodies of VHH-12 had binding activity to Flipin CHO cells overexpressing cyno CD16, with EC50 less than 1 nM.

**Table 30 Binding activity of VHH-hFc antibody with FlpinCHO-cyno CD16 cell detected by FACS**

| Designation of antibody | FlpinCHO-cyno CD16 cell | |
|---|---|---|
| | Maximum mean fluorescence intensity | EC50 (nM) |
| VHH12-02 | 16867 | 0.74 |
| VHH12-03 | 18021 | 0.80 |
| VHH12-04 | 18276 | 0.64 |
| VHH12-05 | 18297 | 0.55 |
| VHH12-06 | 18595 | 0.83 |
| VHH12-07 | 18531 | 0.68 |
| VHH12-08 | 18300 | 0.44 |
| VHH-12 | 18417 | 0.35 |
| P2C47 | 20070 | 0.62 |
| NC | 128 | Negative |

### Example 12 Functional identification of human CD16 antibody

### 12.1 Function of human CD16 antibody detected by luciferase report assay

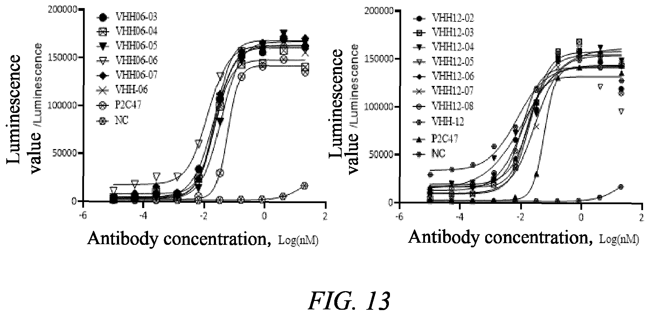

The signalling pathway in the luciferase reporter cell line activated by humanized CD 16 antibodies was detected according to the method of Example 6.1. The results were as shown in Table 31 and FIG. 13. In the report assay, all humanized antibodies could activate the downstream signalling pathway of Jurkat cells, and the activation activity was stronger than that by the P2C47 antibody.

**Table 31 Activation activity by CD16 antibody detected by luciferase report assay**

| Designation of antibody | Activation activity for Jurkat cell | |
|---|---|---|
| | Maximum fluorescence value | EC50 (nM) |
| VHH06-03 | 170610 | 0.02 |
| VHH06-04 | 158520 | 0.02 |
| VHH06-05 | 176000 | 0.03 |
| VHH06-06 | 166815 | 0.01 |
| VHH06-07 | 173270 | 0.02 |
| VHH-06 | 168250 | 0.02 |
| VHH12-02 | 147745 | 0.01 |
| VHH12-03 | 154975 | 0.02 |
| VHH12-04 | 162460 | 0.01 |
| VHH12-05 | 121555 | 0.01 |
| VHH12-06 | 157685 | 0.02 |
| VHH12-07 | 152670 | 0.03 |
| VHH12-08 | 141840 | 0.01 |
| VHH-12 | 142475 | 0.01 |
| P2C47 | 149245 | 0.06 |
| NC | 6395 | 19.04 |

### Example 13 Affinity assay of CD16 antibody

The affinity of humanized CD16 antibodies to human CD16a (158F)-ECD-his proteins was detected according to the method of Example 7.1. The binding rate (Ka), dissociation rate (Kd) and binding affinity (KD) of the antibodies to be detected to human CD16a (158F)-ECD his proteins were as shown in Table 32.

**Table 32 Binding of CD16 antibody to human CD16a protein detected by Biacore**

| Designation of antibody | Human CD16 a (158F) | | |
|---|---|---|---|
| | ka (1/Ms) | kd (1/s) | KD (M) |
| VHH06-03 | 4.48E+04 | 1.29E-03 | 2.87E-08 |
| VHH06-04 | 4.77E+04 | 4.67E-04 | 9.80E-09 |
| VHH06-05 | 4.03E+04 | 1.09E-03 | 2.69E-08 |
| VHH06-06 | 4.71E+04 | 1.16E-03 | 2.46E-08 |
| VHH06-07 | 4.31E+04 | 1.39E-03 | 3.21E-08 |
| VHH12-02 | 3.16E+05 | 2.37E-02 | 7.51E-08 |
| VHH12-03 | 4.38E+05 | 2.44E-02 | 5.57E-08 |
| VHH12-04 | 6.07E+05 | 3.07E-02 | 5.05E-08 |
| VHH12-05 | 3.85E+05 | 2.08E-02 | 5.39E-08 |
| VHH12-06 | 5.92E+05 | 1.64E-02 | 2.76E-08 |
| VHH12-07 | 5.74E+05 | 3.20E-02 | 5.58E-08 |
| VHH12-08 | 6.05E+05 | 2.94E-02 | 4.86E-08 |

## Claims

1. An antibody or an antigen-binding fragment specifically binding to human CD16, **characterised in that** the antibody or the antigen-binding fragment comprises a combination of CDRs; the combination of CDRs comprises CDR1, CDR2 and CDR3; and the CDR1, CDR2 and CDR3 have HCDR1, HCDR2 and HCDR3 selected from a VHH sequence as shown in any one of SEQ ID NOs: 21-34 or SEQ ID NOs: 90-101.

2. The antibody or the antigen-binding fragment of claim 1, **characterised in that** the HCDR1, HCDR2 and HCDR3 are determined according to the Kabat, Chothia or IMGT numbering system; optionally, the HCDR1, HCDR2 and HCDR3 are selected from Table 9;
optionally, the HCDR1 is selected from SEQ ID NO: 49, 52, 54, 57, 60, 62, 65, 66, 67, 68, 70, 72, 78, 79, 80, 81 or 88;
optionally, the HCDR2 is selected from SEQ ID NO: 50, 53, 55, 58, 61, 63, 69, 71, 73, 75, 76, 77, 82 or 85;
optionally, the HCDR3 is selected from SEQ ID NO: 51, 56, 59, 64, 74, 83, 84, 86 or 87;
preferably, according to the Kabat, Chothia or IMGT numbering system, the HCDR1, HCDR2 and HCDR3 are selected from SEQ ID NOs: 49, 50 and 51, SEQ ID NOs: 52, 53 and 51 or SEQ ID NOs: 54, 55 and 56;
preferably, according to the Kabat, Chothia or IMGT numbering system, the HCDR1, HCDR2 and HCDR3 are selected from SEQ ID NOs: 57, 58 and 59, SEQ ID NOs: 60, 61 and 59 or SEQ ID NOs: 62, 63 and 64;
preferably, according to the Kabat, Chothia or IMGT numbering system, the HCDR1, HCDR2 and HCDR3 are selected from SEQ ID NOs: 49, 50 and 51, SEQ ID NOs: 65, 53 and 51 or SEQ ID NOs: 66, 55 and 56;
preferably, according to the Kabat, Chothia or IMGT numbering system, the HCDR1, HCDR2 and HCDR3 are selected from SEQ ID NOs: 49, 50 and 51, SEQ ID NOs: 67, 53 and 51 or SEQ ID NOs: 68, 55 and 56;
preferably, according to the Kabat, Chothia or IMGT numbering system, the HCDR1, HCDR2 and HCDR3 are selected from SEQ ID NOs: 49, 69 and 51, SEQ ID NOs: 70, 71 and 51 or SEQ ID NOs: 72, 73 and 74;
preferably, according to the Kabat, Chothia or IMGT numbering system, the HCDR1, HCDR2 and HCDR3 are selected from SEQ ID NOs: 49, 75 and 51, SEQ ID NOs: 52, 76 and 51 or SEQ ID NOs: 54, 77 and 56;
preferably, according to the Kabat, Chothia or IMGT numbering system, the HCDR1, HCDR2 and HCDR3 are selected from SEQ ID NOs: 49, 50 and 51, SEQ ID NOs: 78, 53 and 51 or SEQ ID NOs: 79, 55 and 56;
preferably, according to the Kabat, Chothia or IMGT numbering system, the HCDR1, HCDR2 and HCDR3 are selected from SEQ ID NOs: 49, 50 and 51, SEQ ID NOs: 80, 53 and 51 or SEQ ID NOs: 81, 55 and 56;
preferably, according to the Kabat, Chothia or IMGT numbering system, the HCDR1, HCDR2 and HCDR3 are selected from SEQ ID NOs: 49, 69 and 51, SEQ ID NOs: 65, 71 and 51 or SEQ ID NOs: 66, 73 and 56;
preferably, according to the Kabat, Chothia or IMGT numbering system, the HCDR1, HCDR2 and HCDR3 are selected from SEQ ID NOs: 57, 82 and 83, SEQ ID NOs: 60, 61 and 83 or SEQ ID NOs: 62, 63 and 84;
preferably, according to the Kabat, Chothia or IMGT numbering system, the HCDR1, HCDR2 and HCDR3 are selected from SEQ ID NOs: 57, 85 and 86, SEQ ID NOs: 60, 61 and 86 or SEQ ID NOs: 62, 63 and 87;
preferably, according to the Kabat, Chothia or IMGT numbering system, the HCDR1, HCDR2 and HCDR3 are selected from SEQ ID NOs: 57, 82 and 86, SEQ ID NOs: 60, 61 and 86 or SEQ ID NOs: 62, 63 and 87;
preferably, according to the Kabat, Chothia or IMGT numbering system, the HCDR1, HCDR2 and HCDR3 are selected from SEQ ID NOs: 88, 82 and 83, SEQ ID NOs: 60, 61 and 83 or SEQ ID NOs: 62, 63 and 84;
and preferably, according to the Kabat, Chothia or IMGT numbering system, the HCDR1, HCDR2 and HCDR3 are selected from SEQ ID NOs: 49, 50 and 51, SEQ ID NOs: 80, 53 and 51 or SEQ ID NOs: 81, 55 and 56.

3. The antibody or the antigen-binding fragment of claim 1 or 2, **characterised in that** the CDR1, CDR2 and/or CDR3 comprise(s) an amino acid sequence with 1, 2 or 3 mutations on the HCDR1, HCDR2 and/or HCDR3; the mutations can be selected from insertion, deletion and/or substitution, and the substitution is preferably a conservative amino acid substitution.

4. The antibody or the antigen-binding fragment of claim 1 or 2, **characterised in that** the CDR1, CDR2 and/or CDR3 comprise(s) a sequence having at least 80%, 85%, 90%, 91%, 92%, 93%, 94%, 95%, 96%, 97%, 98%, 99% or 100% identity to the HCDR1, HCDR2 and/or HCDR3.

5. The antibody or the antigen-binding fragment of any one of claims 1-4, **characterised in that** the antibody or the antigen-binding fragment comprises a single domain antibody, and the single domain antibody comprises the CDR1, CDR2 and CDR3.

6. The antibody or the antigen-binding fragment of claim 5, **characterised in that** the single domain antibody comprises a sequence selected from as shown in any one of SEQ ID NOs: 21-34 or SEQ ID NOs: 90-101;
optionally, the single domain antibody comprises a sequence with at most 20, 19, 18, 17, 16, 15, 14, 13, 12, 11, 10, 9, 8, 7, 6, 5, 4, 3, 2 or 1 mutation relative to the sequence as shown in any one of SEQ ID NOs: 21-34 or SEQ ID NOs: 90-101, the mutation can be selected from insertion, deletion and/or substitution, and the substitution is preferably a conservative amino acid substitution;
and optionally, the single domain antibody comprises a sequence having at least 80%, 85%, 90%, 91%, 92%, 93%, 94%, 95%, 96%, 97%, 98%, 99% or 100% identity to the sequence as shown in any one of SEQ ID NOs: 21-34 or SEQ ID NOs: 90-101.

7. The antibody or the antigen-binding fragment of any one of claims 1-6, **characterised in that** the antibody comprises an FR region in a VHH domain as shown in any one of SEQ ID NOs: 21-34 or SEQ ID NOs: 90-101;
optionally, the antibody comprises a sequence with at most 15, 14, 13, 12, 11, 10, 9, 8, 7, 6, 5, 4, 3, 2 or 1 mutation relative to the FR region in the VHH domain as shown in any one of SEQ ID NOs: 21-34 or SEQ ID NOs: 90-101, the mutation can be selected from insertion, deletion and/or substitution, and the substitution is preferably a conservative amino acid substitution;
and optionally, the single domain antibody comprises a sequence having at least 80%, 85%, 90%, 91%, 92%, 93%, 94%, 95%, 96%, 97%, 98%, 99% or 100% identity to the FR region sequence in the VHH domain as shown in any one of SEQ ID NOs: 21-34 or SEQ ID NOs: 90-101.

8. The antibody or the antigen-binding fragment of any one of claims 1-7, **characterised in that** the antibody or the antigen-binding fragment is: (1) a chimeric antibody or a fragment thereof; (2) a humanized antibody or a fragment thereof; or (3) a fully human antibody or a fragment thereof.

9. The antibody or the antigen-binding fragment of any one of claims 1-8, **characterised in that** the antibody or the antigen-binding fragment comprises or does not comprise an antibody heavy chain constant region; optionally, the antibody heavy chain constant region can be selected from humans, llamas, mice, rats, rabbits or goats; optionally, the antibody heavy chain constant region can be selected from IgG, IgM, IgA, IgE or IgD, and the IgG can be selected from IgG1, IgG2, IgG3 or IgG4; optionally, the heavy chain constant region can be selected from an Fc region, a CH3 region or a complete heavy chain constant region; preferably, the heavy chain constant region is a human Fc region, and preferably comprises a sequence as shown in any one of SEQ ID NOs: 35-48 or SEQ ID NOs: 102-113; and preferably, the antibody or the antigen-binding fragment is a heavy chain antibody.

10. The antibody or the antigen-binding fragment of any one of claims 1-9, **characterised in that** the antibody or the antigen-binding fragment is further coupled with a therapeutic agent or a tracer; preferably, the therapeutic agent is selected from a radioisotope, a cytotoxic agent, a chemotherapeutic drug or an immunomodulator, and the tracer is selected from a radiological contrast agent, a paramagnetic ion, a metal, a fluorescent tag, a chemiluminescence tag, an ultrasound contrast agent and a photosensitizer.

11. The antibody or the antigen-binding fragment of any one of claims 1-10, **characterised in that** the antibody or the antigen-binding fragment specifically binds to human CD16 or cyno CD 16, and preferably, the KD of the antibody or the antigen-binding fragment to the human CD16 or the cyno CD16 is less than 1E-6M, 1E-7M, 2E-7M, 3E-7M, 4E-7M, 5E-7M, 6E-7M, 8E-7M, 9E-7M, 1E-8M, 2E-8M, 3E-8M, 4E-8M, 5E-8M, 6E-8M, 8E-8M, 9E-8M or 1E-9M.

12. The antibody or the antigen-binding fragment of any one of claims 1-11, **characterised in that** the antibody or the antigen-binding fragment binds to CD16A, and does not bind to CD16B or binds weakly to CD16B, and the CD16B is selected from CD16B (NA1), CD16B (NA2) or CD16B (HS).

13. The antibody or the antigen-binding fragment of any one of claims 1-12, **characterised in that** the antibody or the antigen-binding fragment is also linked to other functional molecules, and preferably, the other functional molecules can be selected from one or more of the following: a signal peptide, a protein tag, a cytokine, an angiogenesis inhibitor or an immune checkpoint inhibitor.

14. The antibody or the antigen-binding fragment of claim 13, **characterised in that** the cytokine can be IL-2, IL-6, IL-12, IL-15, IL-21, IFNγ or TNFα; the angiogenesis inhibitor can be endostatin; and the immune checkpoint inhibitor can be SIRPα.

15. A multispecific antigen-binding molecule, **characterised in that** the multispecific antigen-binding molecule comprises the antibody or the antigen-binding fragment of any one of claims 1-14, and an antigen-binding molecule binding to an antigen other than CD16 or binding to a different CD16 epitope than that of the antibody or the antigen-binding fragment of any one of claims 1-14; optionally, the antigen other than CD16 can be selected from: CD137, CD258, PD-1, PD-L1, 4-1BB, CD40, CD64, EGFR, VEGF, HER2, HER1, HER3, IGF-1R, phosphatidylserine (PS), C-Met, BCMA, HSA, GPRC5D, MSLN, a blood brain barrier receptor, GPC3, PSMA, CD33, GD2, ROR1, ROR2, FRα or Gucy2C;
preferably, the other antigen-binding molecule is an antibody or an antigen-binding fragment;
preferably, the multispecific antigen-binding molecule can be bispecific, trispecific or tetraspecific;
and preferably, the multispecific binding molecule can be bivalent, tetravalent or hexavalent.

16. An isolated nucleic acid fragment, **characterised in that** the nucleic acid fragment encodes the antibody or the antigen-binding fragment of any one of claims 1-14 or the multispecific antigen-binding molecule of claim 15.

17. A vector, **characterised in that** the vector comprises the nucleic acid fragment of claim 16.

18. A host cell, **characterised in that** the host cell comprises the vector of claim 17; and preferably, the host cell is a prokaryotic cell or a eukaryotic cell, such as a bacterial (*Escherichia coli*) cell, a fungal (yeast) cell, an insect cell or a mammalian cell (a CHO cell line or a 293T cell line).

19. A method for preparing the antibody or the antigen-binding fragment of any one of claims 1-14 or the multispecific antigen-binding molecule of claim 15, **characterised in that** the method comprises culturing the cell of claim 18, and isolating the antibody, the antigen-binding fragment or the multispecific antigen-binding molecule expressed by the cell.

20. A pharmaceutical composition, **characterised in that** the pharmaceutical composition comprises the antibody or the antigen-binding fragment of any one of claims 1-14, the multispecific antigen-binding molecule of claim 15, the nucleic acid fragment of claim 16, the vector of claim 17 or a product prepared according to the method of claim 19; optionally, the pharmaceutical composition also comprises a pharmaceutically acceptable carrier, a diluent or an auxiliary agent; and optionally, the pharmaceutical composition further comprises an additional anti-tumour agent.

21. A method for treating tumours or cancers, inflammatory diseases or allergies, **characterised in that** the method comprises administering to a subject an effective amount of the antibody or the antigen-binding fragment of any one of claims 1-14, the multispecific antigen-binding molecule of claim 15, the nucleic acid fragment of claim 16, the vector of claim 17, a product prepared according to the method of claim 19 or the pharmaceutical composition of claim 20; and preferably, the tumours or cancers are selected from non-Hodgkin's lymphoma, chronic lymphocytic leukaemia, Hodgkin disease, minimal residual disease and metastatic tumour.

22. The antibody or the antigen-binding fragment of any one of claims 1-14, the multispecific antigen-binding molecule of claim 15, the nucleic acid fragment of claim 16, the vector of claim 17, a product prepared according to the method of claim 19 or the pharmaceutical composition of claim 20 for the use of treating tumours or cancers, inflammatory diseases or allergies; preferably, the tumours or cancers are selected from non-Hodgkin's lymphoma, chronic lymphocytic leukaemia, Hodgkin disease, minimal residual disease and metastatic tumour.

23. A kit, **characterised in that** the kit comprises the antibody or the antigen-binding fragment of any one of claims 1-14, the multispecific antigen-binding molecule of claim 15, the nucleic acid fragment of claim 16, the vector of claim 17, a product prepared according to the method of claim 19 or the pharmaceutical composition of claim 20.

24. A method for detecting CD16 expression in a biological sample, **characterised in that** the method comprises contacting the biological sample with the antibody or the antigen-binding fragment of any one of claims 1-14 under conditions that allow the formation of a complex from the antibody or the antigen-binding fragment of any one of claims 1-14 and CD16; and preferably, the method further comprises detecting the formation of the complex, thereby indicating the presence or expression level of CD16 in the sample.

25. Use of the antibody or the antigen-binding fragment of any one of claims 1-14 in the preparation of a reagent for detecting CD16.
